# EUROPEAN PATENT APPLICATION

(11) **EP 4 177 353 A1**
(43) Date of publication of application: **10.05.2023**
(21) Application number: 21837322.3
(22) Date of filing: 17.06.2021
(51) Int. Cl.: C12Q 1/6869, C12M 1/00, G01N 33/50

(54) **BIOPARTICLE ANALYSIS METHOD AND BIOPARTICLE ANALYSIS SYSTEM**

(30) Priority: 06.07.2020 JP 2020116432
(71) Applicant: Sony Group Corporation, Minato-Ku, Tokyo, 108-0075 (JP)
(72) Inventor: MATSUMOTO Masahiro, Tokyo 108-0075 (JP)
(74) Representative: D Young & Co LLP
(86) International application number: PCT/JP2021/023036
(87) International publication number: WO 2022/009642

(57) **Abstract**

An object of the present technology is to improve analysis efficiency in single cell analysis using a barcoding technique.

The present technology provides a bioparticle analysis method including: a capture step of capturing a bioparticle on a surface, on which a molecule including a bioparticle capturing part, a barcode sequence, and a cleavable linker is immobilized via the linker, via the bioparticle capturing part; a cleavage step of cleaving the linker to release the bioparticle from the surface; and an isolation step of isolating the bioparticle into a microspace. Furthermore, the present technology also provides a bioparticle analysis system that performs the bioparticle analysis method.

## Description

### TECHNICAL FIELD

The present technology relates to a bioparticle analysis method and a bioparticle analysis system. More specifically, the present technology relates to a bioparticle analysis method including a step of isolating a bioparticle into a microspace to analyze components of the bioparticle, and a system that performs the method.

### BACKGROUND ART

As a method for performing single cell analysis, a barcoding technique using a DNA sequence has been proposed. Examples of the barcoding technique include a method using wells and a method using an emulsion.

An example of the method using wells is described in Non-Patent Document 1 below. In the large-scale parallel polymerase cloning method described in Non-Patent Document 1, single cells are randomly placed in wells of several hundred to several thousand nanoliters, and then, their genetic materials are simultaneously amplified for shotgun sequencing. In addition, an example of the method using an emulsion is described in Non-Patent Document 2 below. Non-Patent Document 2 describes a droplet-based microfluidic protocol for highthroughput analysis and sorting of single cells.

### CITATION LIST

### NON-PATENT DOCUMENT

Non-Patent Document 1: Jeff Gole et al., Massively parallel polymerase cloning and genome sequencing of single cells using nanoliter microwells, Nature Biotechnology, Volume 31, Number 12, pages 1126-1132, December 2013
Non-Patent Document 2: Linas Mazutis et al., Single-cell analysis and sorting using droplet-based microfluidics, nature protocols, Volume 8, Number 5, pages 870-891, May 2013

### SUMMARY OF THE INVENTION

### PROBLEMS TO BE SOLVED BY THE INVENTION

For example, the above-described barcoding technique using wells may take time in analyzing cells, for example, because processing for putting one cell into one well takes time. Therefore, it is desirable to improve analysis efficiency in the barcoding technique using wells. In addition, the above-described barcoding technique using an emulsion, there is a low probability that one cell and one barcode are put into one emulsion particle. Therefore, there has been a demand for improving analysis efficiency in the barcoding technique using an emulsion as well.

At this point, an object of the present technology is to improve analysis efficiency in single cell analysis using a barcoding technique.

### SOLUTIONS TO PROBLEMS

The present inventor has found that analysis efficiency can be improved by isolating each cell into a microspace after a barcode is assigned to each cell.

That is, the present technology provides a bioparticle analysis method including:
a capture step of capturing a bioparticle on a surface, on which a molecule including a bioparticle capturing part, a barcode sequence, and a cleavable linker is immobilized via the linker, via the bioparticle capturing part;
a cleavage step of cleaving the linker to release the bioparticle from the surface; and
an isolation step of isolating the bioparticle into a microspace.

In the capture step, a plurality of molecules bound to one bioparticle may have the same barcode sequence.

In an embodiment of the present technology, the bioparticle analysis method may further include a disruption step of disrupting the bioparticle in the microspace.

In the disruption step, the molecule may be dissociated from the bioparticle.

In an embodiment of the present technology, the molecule may further include a target substance capturing part, and
in the disruption step, a target substance constituting the bioparticle or a target substance bound to the bioparticle may be captured by the target substance capturing part.

In an embodiment of the present technology, the bioparticle analysis method may further include an analysis step of analyzing the target substance after the disruption step.

In the analysis step, the barcode sequence may be associated with the target substance.

The target substance may have a base sequence, and
in the analysis step, sequencing processing may be performed on the base sequence of the target substance.

In the cleavage step, a bioparticle to be released from the surface may be selected on the basis of a label of the bioparticle or a label of the molecule.

In the cleavage step, the linker may be cleaved by chemical stimulation or photic stimulation.

In the cleavage step, the captured state of the bioparticle may be maintained by the bioparticle capturing part.

In the cleavage step, only a selected bioparticle may be released from the surface.

In an embodiment of the present technology, the microspace may be a space in an emulsion particle or a space in a well.

In an embodiment of the present technology, the bioparticle analysis method may further include a determination step of determining whether to isolate a bioparticle released from the surface in the cleavage step into a microspace.

In the determination step, the determination may be performed on the basis of light generated by irradiating the bioparticle with light.

In the capture step, the bioparticle and the bioparticle capturing part may be bound to each other in a specific or non-specific manner.

The capture step may include an incubation step for binding the bioparticle and the bioparticle capturing part to each other.

Furthermore, the present technology also provides a bioparticle analysis system including:
a capture kit configured to capture a bioparticle, the capture kit having a surface on which a molecule including a bioparticle capturing part, a barcode sequence, and a cleavable linker is immobilized via the linker;
a linker cleavage device that cleaves the linker to release the bioparticle from the surface; and
an isolation device that forms or has a microspace into which the bioparticle released from the surface is isolated.

The bioparticle analysis system may further include an analysis device that analyzes a target substance constituting the bioparticle or a target substance bound to the bioparticle.

### BRIEF DESCRIPTION OF DRAWINGS

Fig. 1 is an example of a flowchart of an analysis method according to the present technology.
Fig. 2 is a schematic diagram for explaining an operation in each step included in the analysis method according to the present technology.
Fig. 3 is a schematic view illustrating an analysis surface used in the analysis method according to the present technology and molecules immobilized on the surface.
Fig. 4 is a schematic diagram for explaining a particle isolation step.
Fig. 5 is a schematic diagram for explaining the particle isolation step.
Fig. 6 is a diagram illustrating an example of a microchannel used in the particle isolation step.
Fig. 7 is a diagram illustrating an example of a nucleic acid-bound antibody.
Fig. 8A is a diagram illustrating an example of a bioparticle sorting device used in the particle isolation step.
Fig. 8B is a schematic view illustrating how emulsion particles are formed and bioparticles are isolated into the formed emulsion particles in a microchip.
Fig. 9 is an enlarged view of a particle sorting part included in the bioparticle sorting device.
Fig. 10 is a diagram illustrating an example of a block diagram of a control unit included in the bioparticle sorting device.
Fig. 11 is an example of a flowchart of processing by the bioparticle sorting device.
Fig. 12A is an enlarged view of the vicinity of a connection channel included in the bioparticle sorting device.
Fig. 12B is an enlarged view of the vicinity of the connection channel included in the bioparticle sorting device.
Fig. 13A is an enlarged view of the vicinity of a connection channel included in the bioparticle sorting device.
Fig. 13B is an enlarged view of the vicinity of the connection channel included in the bioparticle sorting device.
Fig. 14 is a schematic view of the bioparticle sorting device to which a collection container is connected.
Fig. 15 is a copy of a photograph showing that an emulsion particle was formed by the bioparticle sorting device.
Fig. 16 is a diagram illustrating another example of the microchip.

### MODE FOR CARRYING OUT THE INVENTION

Hereinafter, preferred embodiments for carrying out the present technology will be described. Note that the embodiments to be described below are representative embodiments of the present technology, and the scope of the present technology is not limited only to these embodiments. Note that the present technology will be described in the following order.
1. First Embodiment (Bioparticle Analysis Method)
   (1) Details of Problems of Invention
   (2) Description of First Embodiment
   (3) Example of First Embodiment
      (3-1) Preparation Step
      (3-2) Capture Step
      (3-3) Cleavage Step
      (3-4) Isolation Step
      (3-5) Disruption Step
      (3-6) Analysis Step
2. Second Embodiment (Bioparticle Analysis System)
3. Third Embodiment (Surface)

### 1. First Embodiment (Bioparticle Analysis Method)

### (1) Details of Problems of Invention

As described above, in order to perform single cell analysis, a barcoding technique using a DNA sequence has been proposed. This method makes it possible to detect a large number of molecules (e.g., cell surface antigens, intracellular proteins, and mRNAs). In this method, one cell and one bar coding bead (or gel bead) are put in a microspace, e.g., a well or an emulsion, and then, the cell is lysed, such that a barcode may be assigned to a molecule to be measured.

The analysis using this method may be performed, for example, as follows. That is, different barcode molecules are present in respective microspaces. Then, cells are put into the microspaces, and thereafter, the cells are lysed and incubated, such that the barcode molecule is bound to a molecule to be measured in each microspace to form a barcoded molecule to be measured. Then, all the barcoded molecules to be measured are mixed, and sequences of the molecules to be measured are decrypted by an analysis device, e.g., a next-generation sequencing (NGS). On the basis of barcode information included in the decryption result, it is possible to identify, for example, which cell a certain mRNA is derived from, to perform single cell analysis. Here, the analysis method using the next-generation sequencer is capable of detection even in a case where the number of markers is 10² or more, for example, because there is no spectral overlap which is a disadvantage of a fluorescence detection method (e.g., flow cytometry or fluorescence imaging).

Analysis efficiency in single cell analysis using a barcoding technique depends on a probability that one cell and one barcode bead (or gel bead) are encapsulated in one microspace. In addition, the analysis efficiency may decrease due to various factors that are peculiar to an analysis method.

For example, in a well-based method, a cell sorter is used to place one cell in one well, or a smaller number of cells than the number of wells are seeded in a well plate. Thereafter, single cell barcoding is performed by placing a barcode bead (e.g., gel bead). In order to selectively acquire a sample capable of single cell analysis, for example, a sample may be taken by a micropipette from a well satisfying the condition that one cell and one barcode bead (or gel bead) are contained in one well, or alternatively, a sample derived from a well that does not satisfy the condition may be excluded. However, such processing is time consuming and inefficient. As another method, analysis is performed by a next-generation sequencer without selectively acquiring a sample capable of single cell analysis, and then, data derived from a sample that is not a single cell is excluded, but this leads to a waste in the analysis. Furthermore, in a case where a single cell is sorted on a well plate using a cell sorter on the basis of a detection signal, for example, the number of wells in the well plate is limited, resulting in a low analysis scale and throughput. Moreover, since sorting cannot be performed while a sorting nozzle moves between the wells, the target cells flowing during this time are lost.

On the other hand, an emulsion-based method is easier in increasing an analysis scale than the well-based method. As an example of a method for producing an emulsion, a solution containing particles to be captured (e.g., a hydrophilic solution) and a solution immiscible therewith (e.g., a hydrophobic solution) are caused to flow through a microchannel, and the hydrophilic solution is captured in the hydrophobic solution using a shear force. Alternatively, the hydrophilic solution may be captured in the hydrophobic solution by changing a flow rate of each solution while the solutions flow. For example, an emulsion may be formed in the microchannel, and then, particles and barcode beads may be captured in emulsion particles.

In the emulsion-based method, in many cases, the hydrophilic solution is incorporated into the hydrophobic solution to form emulsion particles at a regular timing, and particles and barcode beads incorporated into the emulsion particles are contained in the hydrophilic solution in a diluted state at a predetermined concentration. This is to avoid formation of doublet in which two cells or two barcode beads are captured in one emulsion particle. Accordingly, in many cases, cells or barcode beads are not captured in the emulsion particles, and the emulsion particles are often empty. In the emulsion-based method, a chance of success in capturing a cell in an emulsion particle is not high, and it has been said that the chance of success in capturing one cell in one emulsion particle is about 65% at the maximum. A probability that one barcode bead is captured in addition to one cell in one emulsion particle is further reduced.

A probability that no, one, or two or more cells are put in one emulsion particle basically follows a Poisson distribution (see Non-Patent Document 2 above). In a case where the number of cells in a sample for single cell analysis is small, particularly in a clinical sample from which only 10⁴ to 10⁵ cells can be obtained or a rare cell sample such as CTC, it is important to efficiently capture one cell and one barcode bead or one gel bead in one emulsion.

Furthermore, in the emulsion-based method, in a case where two or more cells are put in one emulsion particle, the emulsion particle is not a target for single cell analysis. Thus, even if the emulsion particle is subjected to analysis processing (e.g., next-generation sequencing), analysis data thereof is excluded. Therefore, the formation of doublet leads to a waste of analysis.

Furthermore, before emulsification, a target cell group to be subjected to single cell analysis may be sorted and purified using a cell sorter or the like. However, in this case, a device called a cell sorter is required in addition to the emulsion forming device, and a workflow called cell sorting is also added. Therefore, costs increase in terms of time and expense.

Based on the above, an object of the present technology is to provide a method for improving analysis efficiency in single cell analysis using a barcode sequence.

### (2) Description of First Embodiment

A bioparticle analysis method according to the present technology includes: a capture step of capturing a bioparticle on a surface, on which a molecule including a bioparticle capturing part, a barcode sequence, and a cleavable linker is immobilized via the linker, via the bioparticle capturing part; a cleavage step of cleaving the linker to release the bioparticle from the surface; and an isolation step of isolating the bioparticle into a microspace. By performing these steps, the barcode sequence and the bioparticle can be efficiently isolated into one microspace. As a result, for example, a target substance contained in the bioparticle can be efficiently bound to the barcode sequence, and furthermore, each bioparticle can be efficiently analyzed. In this way, according to the present technology, efficiency in single cell analysis using a barcode sequence can be improved.

In a preferred embodiment of the present technology, the bioparticle analysis method may further include a disruption step of disrupting the bioparticle in the microspace. As a result, components contained in the bioparticle can be efficiently bound to the barcode sequence, thereby improving efficiency in analyzing the bioparticle.

In a preferred embodiment of the present technology, the bioparticle analysis method may further include an analysis step of analyzing a target substance contained in the bioparticle. The target substance may be, for example, a substance bound to the molecule containing the barcode sequence. The target substance may be a nucleic acid, for example, an RNA or a DNA, particularly an mRNA. By using the barcode sequence, a result of analyzing the target substance can be associated with the bioparticle, thereby making it possible to analyze a single cell.

### (3) Example of First Embodiment

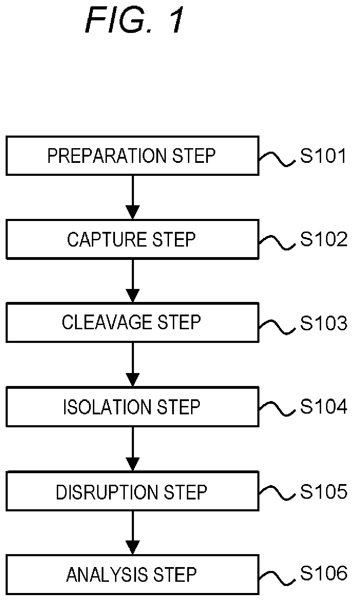

Fig. 1 illustrates an example of a flowchart of a bioparticle analysis method according to the present technology. As illustrated in Fig. 1, the analysis method according to the present technology may include a preparation step S101, a capture step S102, a cleavage step S103, an isolation step S104, a disruption step S105, and an analysis step S106. These steps will be described below.

### (3-1) Preparation Step

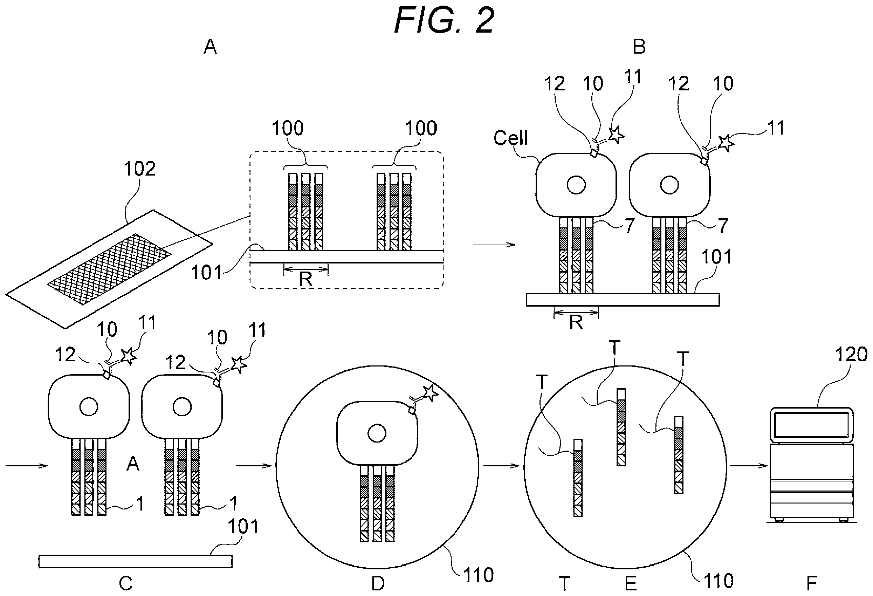

In the preparation step S101, a surface on which a molecule including a bioparticle capturing part, a barcode sequence, and a cleavable linker is immobilized via the linker is prepared. For example, as illustrated in Fig. 2A, an analysis substrate (e.g., a slide glass) 102 having a surface 101 on which a plurality of molecules 100 is immobilized is prepared.

The molecule 100 includes a bioparticle capturing part, a barcode sequence, and a cleavable linker. In the present specification, the molecule including a bioparticle capturing part, a barcode sequence, and a linker is a molecule used for capturing a target substance, and may also be referred to as a target capturing molecule. The target capturing molecule is a term for referring to a molecule used in the present technology, and may also be used in the present specification to refer to, for example, the molecule after the target substance is captured or the molecule after the linker is cleaved in the cleavage step to be described later. The target capturing molecule may be, for example, either a single molecule or 08 or a complex molecule. The single molecule may refer to, for example, one type of molecule having a plurality of functions, and may be, for example, one nucleic acid (e.g., a DNA or an RNA) including a nucleic acid part formed as the linker, a nucleic acid part formed as the barcode sequence, and a part formed as the target substance capturing part. The complex molecule may be, for example, a molecular assembly including two or more kinds of molecules (e.g., a conjugate of two or more kinds of molecules), and may be, for example, a conjugate of nucleic acids including a nucleic acid part formed as the linker and a nucleic acid part formed as the barcode sequence and polypeptides (e.g., proteins or parts thereof, or oligopeptides) formed as the target substance capturing part.

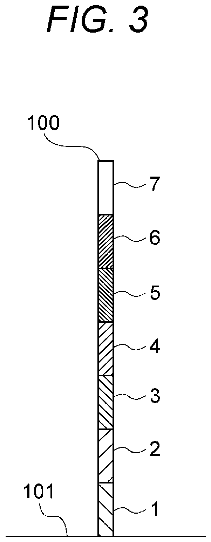

An example of a structure of the molecule 100 will be described with reference to Fig. 3. The molecule 100 illustrated in Fig. 3 includes a linker 1, a collection sequence part 2, an amplification sequence part 3, a barcode sequence part 4, a unique molecular identifier (UMI) part 5, a target substance capturing part 6, and a bioparticle capturing part 7. The molecule 100 is immobilized on the surface 101 via the linker 1.

The collection sequence part 2, the amplification sequence part 3, the barcode sequence part 4, and the UMI part 5 may be formed as a series of nucleic acids (particularly DNAs). When the target substance capturing part 6 is a nucleic acid, the target substance capturing part 6 may also be formed together with the collection sequence part 2, the amplification sequence part 3, the barcode sequence part 4, and the UMI part 5 as a series of nucleic acids (particularly DNAs). In such a case, for example, one end close to the immobilized portion between the surface 101 and the molecule 100 may be a 5' end, and the other end may be a 3' end.

The components of the molecule 100 will be described below.

The linker 1 may be a linker cleavable by stimulation, and is, for example, a linker cleavable by photic stimulation or chemical stimulation. The photic stimulation is suitable particularly for selectively stimulating a specific position in the cleavage step to be described later.

For example, the linker 1 may include any one selected from an arylcarbonylmethyl group, a nitroaryl group, a coumarin-4-ylmethyl group, an arylmethyl group, a metal containing group, and other groups as a linker cleavable by photic stimulation. As such a group, what is described in, for example, Photoremovable Protecting Groups in Chemistry and Biology: Reaction Mechanisms and Efficacy, Chem. Rev. 2013, 113, 119-191 may be used.

For example, the arylcarbonylmethyl group may be a phenacyl group, an o-alkylphenacyl group, or a p-hydroxyphenacyl group. The nitroaryl group may be, for example, an o-nitrobenzyl group, an o-nitro-2-phenethyloxycarbonyl group, or o-nitroanilide. The arylmethyl group may be, for example, one into which a hydroxy group is introduced, or one into which no hydroxy group is introduced.

In a case where the linker 1 is a linker cleavable by photic stimulation, the linker may preferably be cleaved by light having a wavelength of 360 nm or more. The linker may be a linker that is preferably cleaved at an energy of 0.5µJ/µm² or less. (Light-sheet fluorescence microscopy for quantitative biology, Nat Methods. 2015 Jan; 12(1): 23-6. doi: 10.1038/nmeth.3219.). By adopting a linker that is cleaved by light having the wavelength described above or at the energy described above, it is possible to reduce cell damage (particularly, cleavage of DNA or RNA) that may occur when photic stimulation is applied.

More preferably, the linker may be a linker cleaved by light in a short wavelength region, particularly light in a wavelength region of 360 nm to 410 nm, or may be a linker cleaved by light in a near infrared region or an infrared region, particularly light in a wavelength region of 800 nm or more. In a case where the linker is a linker that is efficiently cleaved by light having a wavelength in a visible light region, it may be difficult to handle the analysis surface. Therefore, the linker is preferably a linker cleaved by the light in the short wavelength region described above or the light in the near infrared region or the infrared region described above.

The linker 1 may include, for example, a disulfide bond or a restriction endonuclease recognition sequence as a linker cleavable by chemical stimulation. In order to cleave the disulfide bond, for example, a reducing agent such as tris(2-carboxyethyl)phosphine (TCEP), dithiothreitol (DTT), or 2-mercaptoethanol is used. For example, in a case where TCEP is used, a reaction is performed at 50 mM for about 15 minutes. In order to dissociate the restriction endonuclease recognition sequence, an appropriate restriction endonuclease (http://catalog.takara-bio.co.jp/product/basic_info.php?unitid=U100003632) is used for each sequence. 1 U of restriction endonuclease activity is an amount of endonuclease for completely degrading 1 µg of λDNA for one hour at 37°C in principle in 50 µl of each endonuclease reaction solution, and the amount of the endonuclease is adjusted according to an amount of the restriction endonuclease recognition sequence.

In order to increase efficiency in the cleavage step to be described later, the molecule 100 may include a plurality of cleavable linkers. Preferably, the plurality of linkers may be connected to one another in series. For example, in a case where one linker has a cleavage probability of 0.8, the cleavage probability is improved to 0.992(=1-0.2³) by connecting three linkers to one another in series.

The collection sequence part 2 includes a nucleic acid used for collecting a molecule 100 released from a bioparticle when the bioparticle is disrupted in the disruption step to be described later. The nucleic acid may be a DNA or an RNA, and is particularly a DNA. Note that, for the collection, a bead on which a nucleic acid complementary to the nucleic acid is immobilized may be used. Such a bead makes it possible to efficiently collect the molecule 100 having the collection sequence part 2. A base sequence of the nucleic acid included in the collection sequence part 2 may be appropriately set by a person skilled in the art.

The amplification sequence part 3 may include, for example, a nucleic acid having a primer sequence used for amplifying a nucleic acid or a promoter sequence used for transcribing a nucleic acid in the analysis step to be described later. The nucleic acid may be a DNA or an RNA, and is particularly a DNA. The amplification sequence part 3 may have both a primer sequence and a promoter sequence. The primer sequence may be, for example, a PCR handle. The promoter sequence may be, for example, a T7 promoter sequence.

The barcode sequence part 4 includes a nucleic acid having a barcode sequence. The nucleic acid may be particularly a DNA or an RNA, and more particularly a DNA. The barcode sequence may be used, for example, to identify a captured bioparticle (particularly a cell or an exosome), and particularly, may be used as an identifier for distinguishing a bioparticle isolated into one microspace from a bioparticle isolated into another microspace. In addition, the barcode sequence may be used as an identifier for distinguishing a target capturing molecule including one barcode sequence from a target capturing molecule including another barcode sequence. The barcode sequence may be associated with a bioparticle to which the target capturing molecule including the barcode sequence is bound. In addition, the barcode sequence may be associated with a microspace in which the bioparticle to which the target capturing molecule including the barcode sequence is bound is isolated, and furthermore, may be associated with information regarding a position of the microspace (hereinafter also referred to as "position information"). The position information may be for identifying a position on the surface 101, and is, for example, information regarding XY coordinates, but is not limited thereto. An ID number may be assigned to the barcode sequence associated with the position information. The ID number may be used in the steps subsequent to the cleavage step. The ID number may correspond to the barcode sequence on a one-to-one basis, and may be used as data corresponding to the barcode sequence in the steps subsequent to the cleavage step.

A plurality of target capturing molecules immobilized within a certain region of the surface 101 may have the same barcode sequence. As a result, the certain region and the barcode sequence are associated with each other. By setting a size of the certain region to be smaller than a size of a bioparticle, the target capturing molecule including the barcode sequence can be associated with a position at which one bioparticle is present. For example, as illustrated in A and B of Fig. 2, a region R in which a plurality of target capturing molecules 100 including the same barcode sequence is immobilized may be smaller than a size of a bioparticle (denoted as a cell in Fig. 2).

In this way, the surface used in the bioparticle analysis method according to the present technology may have a plurality of regions in each of which a plurality of target capturing molecules having the same barcode sequence is immobilized. The barcode sequence may be different for each region. A size of each region (e.g., a diameter, a long diameter, a length of a long side, or the like as a maximum dimension of the region) may preferably be smaller than a size of a bioparticle, for example, 50 um or less, preferably 10 um or less, and more preferably 5 um or less.

The plurality of regions may be arranged at an interval, for example, such that a bioparticle captured by a target capturing molecule immobilized in one region is not captured by a target capturing molecule immobilized in another region. The interval may be, for example, a distance equal to or larger than the size of the bioparticle, and may be preferably a distance larger than the size of the bioparticle.

The number of a plurality of regions is preferably larger than the number of bioparticles applied to the surface 101 in the capture step. This prevents two bioparticles from being captured in one region.

In an embodiment of the present technology, target capturing molecules whose sequences include a known barcode sequence may be immobilized in a predetermined region. For example, the surface 101 has a plurality of regions, and a plurality of target capturing molecules immobilized in each of the plurality of regions may include the same barcode sequence. The plurality of regions may be set to be smaller than a size of a bioparticle to be captured. The surface 101 configured as described above makes it possible to associate each of the plurality of regions with a barcode sequence included in the plurality of target capturing molecules immobilized in each of the regions.

A region in which target capturing molecules including the same barcode sequence are immobilized as described above is also referred to as a spot in the present specification. That is, a size of the spot may be, for example, 50 um or less, preferably 10 um or less, and more preferably 5 um or less.

The surface 101 configured as described above makes it possible to associate a barcode sequence included in a certain target capturing molecule with a position at which the certain target capturing molecule is present, at a time point when the target capturing molecules are immobilized on the surface 101. For the immobilization, for example, biotin is bound to the linker 1 of the target capturing molecule, and streptavidin is bound to the surface 101 on which the target capturing molecule is immobilized, such that the biotin and the streptavidin are bound to each other, thereby immobilizing the target capturing molecule on the surface 101.

In another embodiment of the present technology, target capturing molecules each including a barcode sequence may be randomly arranged on the surface 101.

In this case, after the target capturing molecules each including a barcode sequence are immobilized on the surface 101, a barcode sequence included in a certain target capturing molecule is associated with a position at which the certain target capturing molecule is present by reading the barcode sequence included in the immobilized target capturing molecule. The reading can be performed by a method such as sequencing by synthesis, sequencing by ligation, or sequencing by hybridization.

Furthermore, a barcode sequence included in a certain target capturing molecule may not be associated with a position at which the certain target capturing molecule is present. Since a bioparticle and a target capturing molecule are isolated from each other into a microspace by the isolation step to be described later, the bioparticle and the target capturing molecule (particularly a barcode sequence included in the target capturing molecule) are associated with each other on a one-to-one basis.

In this embodiment, for example, a bead (e.g., gel bead) to which a plurality of target capturing molecules including the same barcode sequence is bound may be used, and the bead (e.g., gel bead) may be immobilized on the surface 101. A size of the bead (e.g., gel bead) may be, for example, 50 um or less, preferably 10 um or less, and more preferably 5 um or less. In order to bind a target capturing molecule to a bead (e.g., gel bead), for example, a combination of biotin and streptavidin may be used. For example, biotin is bound to the linker 1 of the target capturing molecule, and streptavidin is bound to the bead, such that the biotin and the streptavidin are bound to each other, thereby immobilizing the target capturing molecule on the bead.

The surface 101 may have a plurality of recesses therein. In the above-described embodiment, one spot or one bead may be arranged in each of the plurality of recesses. The spots or the beads can be more easily arranged on the surface 101 through the plurality of recesses. For example, the recess preferably has a size in which one bead is placed. The recess may have a circular shape, an elliptical shape, a hexagonal shape, or a quadrangular shape, but the shape of the recess is not limited thereto.

Furthermore, some portions of the surface 101, where the spots or the beads are arranged, may be in a different state from the other portions of the surface 101. For example, some surface portions where the spots or the beads are arranged may be hydrophilic, and the other surface portions may be hydrophobic, or the other surface portions may be hydrophobic while each having a protrusion. As an example of a method for imparting hydrophilicity to the surface, reactive ions may be etched in the presence of oxygen, or deep ultraviolet light may be irradiated in the presence of ozone. In such a method, a mask may be used with through holes formed in the portions to which hydrophilicity is to be imparted. In addition, as an example of a method for imparting hydrophobicity to the surface, a spray-onsilicone, for example, Techspray 2101-12S, may be used. In order to impart hydrophobicity, for example, a mask may also be used with through holes formed in the portions to which hydrophobicity is to be imparted.

For example, the target capturing molecule can also be synthesized on the substrate using a DNA microarray preparation technique or the like. For example, a target capturing molecule can be synthesized at a specific position using a technology such as a digital micromirror device (DMD), a liquid crystal shutter, or a spatial light phase modulator, which is used for photolithography. A method for the synthesis is described, for example, in Basic Concepts of Microarrays and Potential Applications in Clinical Microbiology, CLINICAL MICROBIOLOGY REVIEWS, Oct. 2009, p. 611-633. Note that, in a case where a target capturing molecule is synthesized on the substrate by the above-described synthesis method, information on a position at which the target capturing molecule is synthesized is acquired when the target capturing molecule is synthesized, and a barcode sequence is associated with the position information. At that time, an ID number may be assigned.

In an embodiment of the present technology, all of the target capturing molecules immobilized on the surface may include a common oligo sequence. By using a nucleic acid that is fluorescently labeled while having a sequence complementary to the oligo sequence, it is possible to confirm a position at which a target capturing molecule is immobilized (particularly a position of a spot or a position of a bead), particularly in a dark field. In addition, in a case where there is no recess or protrusion described above on the surface, it may be difficult to grasp the position at which the target capturing molecule is immobilized. In this case, the fluorescent label makes it easy to grasp the position at which the target capturing molecule is immobilized.

The UMI part 5 may include a nucleic acid, particularly a DNA or an RNA, and more particularly a DNA. The UMI part 5 may have a sequence of, for example, 5 to 30 bases, particularly 6 to 20 bases, and more particularly 7 to 15 bases.

The UMI part 5 may be formed to have a different sequence between target capturing molecules immobilized on the surface 101. For example, in a case where the UMI part has a nucleic acid sequence of 10 bases, the number of types of UMI sequences is 4 to the power of 10, that is, one million or more.

The UMI part 5 may be used to quantify a target substance. For example, in a case where the target substance is an mRNA, a UMI sequence may be added to a cDNA obtained by reversely transcribing the mRNA as the target substance, for example, in the analysis step to be described later. A large number of cDNAs obtained by amplifying a cDNA obtained by reverse transcription from one mRNA molecule have the same UMI sequence, but a large number of cDNAs obtained by amplifying a cDNA obtained by transcription from another mRNA molecule having the same sequence as the one mRNA molecule have a different UMI sequence from those from the one mRNA molecule. Therefore, the number of copies of mRNA can be determined by counting the number of types of UMI sequences having the same cDNA sequence.

For example, the UMI part 5 may be formed to have a different sequence between a plurality of target capturing molecules including the same barcode sequence while being immobilized in one region R (e.g., the spot or the bead). That is, the plurality of target capturing molecules immobilized in the region R (e.g., the spot or the bead) may have different UMIs while having the same barcode sequence.

The target substance capturing part 6 includes a component for capturing a molecule contained in a bioparticle. The component may be, for example, a nucleic acid or a protein. In a case where the component is a nucleic acid, the nucleic acid may have, for example, a poly T sequence in order to comprehensively capture mRNAs contained in a cell. Alternatively, the nucleic acid may have a sequence complementary to the target sequence. In a case where the component is a protein, the protein may be, for example, an antibody. The component may be an aptamer or a molecular imprinted polymer.

The target substance capturing part 6 may include two or more kinds of components for capturing a molecule contained in a cell. The target substance capturing part 6 may include both a protein and a nucleic acid, and may include, for example, both an antibody and a poly T sequence. This makes it possible to simultaneously detect both a protein and an mRNA.

The bioparticle capturing part 7 includes a component for capturing a bioparticle, and particularly includes a component for capturing a cell. The component may be, for example, an antibody, an aptamer, or an oleyl group. The antibody may be an antibody that is bound to a constituent (particularly a surface antigen) present on a surface of a bioparticle such as a cell or an exosome. The aptamer may be a nucleic acid aptamer or a peptide aptamer. The aptamer may also be bound to a constituent (particularly a surface antigen) present on a surface of a bioparticle such as a cell or an exosome. The oleyl group may bind a bioparticle including a lipid bilayer membrane such as a cell or an exosome thereto.

The surface 101 may preferably be a surface of a transparent substrate. The substrate may be entirely transparent, or may be transparent only in a portion on which a target capturing molecule is immobilized. The surface of the substrate is preferably planar to bring a specimen in contact therewith in a good condition. The transparent substrate may be, for example, a glass substrate or a resin substrate. The substrate may be, for example, a slide glass. The transparency makes it easy to select a bioparticle to be cleaved in the detection step and the cleavage step to be described later.

The number and density of molecules 100 bound to the surface 101 can be increased, for example, by increasing a surface area of surface 101. Furthermore, the plurality of molecules 100 may be connected to each other in series. In this case, a cleavage condition between the substrate and a molecule 100 is preferably different from a cleavage condition between two molecules 100. When the molecules are cleaved from each other in a state where the substrate and the molecule 100 are also cleaved from each other, the cleaved molecule may be bound to another adjacent bioparticle. However, the different cleavage conditions make it possible to prevent the cleaved molecule from being bound to another adjacent bioparticle. For example, a linker binding a molecule 100 to the substrate may be a linker cleavable by photic stimulation, and a linker binding a molecule 100 to another molecule 100 may be a linker cleavable by chemical stimulation. The opposite is also possible. Alternatively, a linker binding a molecule 100 to the substrate may be a linker cleavable by chemical stimulation, and a linker binding a molecule 100 to another molecule 100 may be a linker cleavable by another type of chemical stimulation. For example, the former may include a restriction endonuclease recognition sequence, and the latter may include another restriction endonuclease recognition sequence. Alternatively, the former may include a disulfide bond, and the latter may include a restriction endonuclease recognition sequence. In addition, molecules may be bound to each other by an amino acid, and may be cleaved from each other in the disruption step to be described later (particularly simultaneously with cytolysis) by a reagent (e.g., proteinase K) used for the cytolysis.

### (3-2) Capture Step

In the capture step S102, for example, as illustrated in Fig. 2B, a bioparticle (a cell in Fig. 2B) is captured by a molecule 100. Particularly, the bioparticle is captured by a bioparticle capturing part 7 of the molecule 100. In the capture step S102, the bioparticle and the bioparticle capturing part 7 may be bound to each other in a specific or non-specific manner.

For example, in a case where the bioparticle is a cell, a surface antigen of the cell may be bound to an antibody or aptamer contained in the bioparticle capturing part 7, such that the cell is captured by the molecule 100. The antibody and the aptamer may be specific or non-specific. Furthermore, in this case, a lipid bilayer membrane of the cell may be bound to an oleyl group contained in the bioparticle capturing part 7, such that the cell is captured by the molecule 100.

Alternatively, for example, in a case where the bioparticle is an exosome, a surface constituent (a component constituting a lipid bilayer membrane) of the bioparticle may be bound to an oleyl group contained in the bioparticle capturing part 7, such that the bioparticle is captured by the molecule 100. Alternatively, in this case, the surface constituent of the exosome may be bound to an antibody or an aptamer contained in the bioparticle capturing part 7, such that the bioparticle is captured by the molecule 100.

The capture step S102 may include an application step of applying a bioparticle to the surface 101. The application step may be performed, for example, in such a manner that a bioparticle-containing sample (e.g., a bioparticle-containing liquid) is brought into contact with the surface 101. For example, the bioparticle-containing sample may be dropped onto the surface 101.

In the capture step S102, preferably, a plurality of molecules bound to one bioparticle may have the same barcode sequence. This makes it possible to associate one barcode sequence with one bioparticle. Furthermore, UMI parts included in the plurality of molecules preferably have different sequences. This makes it possible to determine the number of copies of, for example, mRNA.

In the present technology, a label may be assigned to the bioparticle. The label may be used to select a bioparticle to be released from the surface, for example, in the cleavage step to be described later. The label assigned to the bioparticle is preferably an antibody having a label. For example, an antibody labeled with a fluorochrome (hereinafter, also referred to as a "fluorochrome-labeled antibody") may be bound to the bioparticle. An example of such an antibody is illustrated in Fig. 2B.

As illustrated in Fig. 2B, an antibody 10 bound to (particularly specifically bound to) a surface antigen 12 of a bioparticle may be bound to the antigen. The antibody 10 may be labeled with, for example, a fluorochrome 11. By using the antibody 10, a bioparticle to be cleaved can be selected on the basis of fluorescence generated from the fluorochrome that labels the antibody, for example, in the detection step to be described later. Furthermore, the fluorescence can also be used to determine whether to isolate a bioparticle into a microspace, for example, in the determination step to be described later.

The fluorochrome-labeled antibody may be bound to the bioparticle before the bioparticle is captured on the surface 101, or may be bound to the bioparticle after the bioparticle is captured on the surface 101.

In the present technology, a label may be assigned to a molecule 100. The label may be used to select a bioparticle to be released from the surface, for example, in the cleavage step to be described later. The label assigned to the molecule 100 is, for example, a fluorochrome. For example, some of the nucleic acids constituting the molecule 100 may be nucleic acids labeled with a fluorochrome.

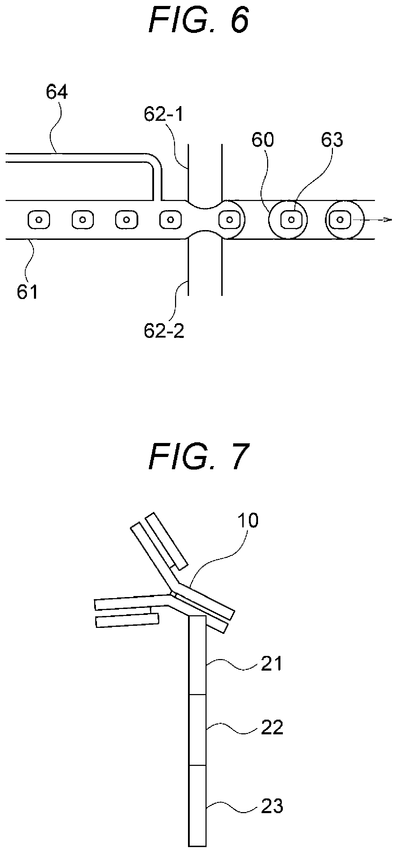

Furthermore, in the present technology, an antibody to which a nucleic acid including an antibody barcode sequence is bound (hereinafter, also referred to as a "nucleic acid-bound antibody") may be bound to a bioparticle. The antibody barcode sequence is a barcode sequence for identifying a nucleic acid-bound antibody. For example, a nucleic acid-bound antibody illustrated in Fig. 7 is bound to the bioparticle instead of or in addition to the fluorochrome-labeled antibody described with reference to Fig. 2B.

The nucleic acid-bound antibody illustrated in Fig. 7 includes an antibody 10 and nucleic acids bound to the antibody. For example, as illustrated in Fig. 7, the nucleic acids include a first nucleic acid 21, a second nucleic acid 22, and a third nucleic acid 23. These nucleic acids may be arranged in the order illustrated in Fig. 7, or may be arranged in another order.

The first nucleic acid 21 may include an amplification primer sequence. Since the first nucleic acid 21 includes an amplification primer sequence, the barcode sequence part 4, the unique molecular identifier (UMI) part 5, and/or the like included in the molecule 100 can be assigned to the second nucleic acid 22 and the third nucleic acid 23 to be described later at the time of amplification. Furthermore, a sequence for sequence processing, e.g., an adapter sequence, or the like can be assigned.

The second nucleic acid 22 may include an antibody barcode sequence. The antibody barcode sequence may be used to distinguish a nucleic acid-bound antibody bound to one bioparticle from a nucleic acid-bound antibody bound to another bioparticle. For example, the antibody barcode sequence may be different for each type of antibody, or the antibody barcode sequence may be different for each type of bioparticle. This makes it possible to identify a bioparticle to which a nucleic acid-bound antibody was bound after the bioparticle is disrupted in the disruption step to be described later.

The third nucleic acid 23 may include a poly A sequence. As a result, after the disruption step to be described later, the nucleic acids including the first nucleic acid 21 and the second nucleic acid 22 as described above can be captured by the poly T sequence included in the target substance capturing part 6 of the molecule 100 via the third nucleic acid 23. Then, a complex of the nucleic acids and the molecule 100 is formed through the capture step. By amplifying the complex, for example, using the first nucleic acid 21, a nucleic acid to which an antibody barcode sequence is assigned as a second nucleic acid 22 is generated in the molecule 100. The nucleic acid generated by the amplification has an antibody barcode sequence, and the antibody barcode sequence is different, for example, for each type of antibody as described above, that is, associated with the type of antibody. Therefore, information regarding the type and/or the number of nucleic acid-bound antibodies is maintained in the form of an antibody barcode sequence in a product of the amplification, and the type and/or the number of nucleic acid-bound antibodies associated with the antibody barcode sequence can be identified, for example, from the sequence and/or the number of nucleic acids having the antibody barcode sequence. This makes it possible to identify the type and the number of nucleic acid-bound antibodies bound to a bioparticle (i.e., a cell or the like). The identification may be performed, for example, in the analysis step to be described later. The sequence analysis of the amplification product for the identification may be performed by, for example, NGS.

The capture step S102 may include an incubation step for binding the bioparticle and the bioparticle capturing part to each other. Incubation conditions such as incubation time and temperature may be determined depending on the type of the bioparticle capturing part to be used.

After the capture step S102, a removal step of removing a bioparticle that has not bound to the molecule 100 may be performed. Furthermore, after the capture step S102, a removal step of removing a substance unnecessary in the cleavage step, such as an antibody that has not bound to the bioparticle, may be performed. The removal step may include washing the surface 101 using a liquid, e.g., a buffer.

### (3-3) Cleavage Step

In the cleavage step S103, the linker 1 is cleaved, and the bioparticle captured in the capture step S102 is released from the surface 101. Preferably, in the cleavage step S103, the captured state of the bioparticle is maintained by the bioparticle capturing part 7. The captured state may be maintained until the bioparticle is completely isolated in the isolation step S104 to be described later, and may be maintained, for example, until the bioparticle is completely disrupted in the disruption step S105 to be described later.

The cleavage may be performed on an entire portion of the surface 101, or may be performed on a partial portion of the surface 101. In the latter case, the partial portion of the surface may be selected, for example, on the basis of a detection result of the detection step to be described below.

Alternatively, the cleavage may be performed to release all of the bioparticles captured on the surface 101 from the surface 101, or may be performed to release some of the bioparticles captured on the surface 101 from the surface 101. In the latter case, some of the bioparticles may be selected, for example, on the basis of a detection result of the detection step to be described below.

For example, the bioparticle to be released from the surface 101 may be selected on the basis of a label of the bioparticle or a label of the molecule 100. The label of the bioparticle may be, for example, a fluorochrome constituting the fluorochrome-labeled antibody described in section (3-2) above, or a label (particularly a fluorochrome) present inside the bioparticle. The label of the molecule 100 may be, for example, a label assigned to the molecule 100 described in section (3-2) above, particularly a fluorochrome.

Note that, in the bioparticle analysis method according to the present technology, the cleavage step may be performed without performing the detection step after the capture step.

In an embodiment of the present technology, the cleavage step S103 may include a detection step of detecting light generated from a bioparticle or light from a substance bound to a bioparticle, and a linker cleavage step of cleaving a linker on the basis of a detection result in the detection step to release the bioparticle from the surface 101. This makes it possible to select a bioparticle to be released from the surface 101, for example, according to the detection result. As a result, unintended bioparticles can be excluded from targets in the analysis step to be described later, thereby improving analysis efficiency.

In another embodiment of the present technology, in the cleavage step S103, the linker cleavage step may be performed without performing the detection step. By omitting the detection step, the number of steps in the analysis method according to the present technology can be reduced.

Hereinafter, the detection step and the linker cleavage step will be individually described.

### (3-3-1) Detection Step

The cleavage step S103 may include a detection step of detecting any one or two or more of light (e.g., scattered light and/or autofluorescence) derived from a bioparticle, light (e.g., fluorescence) derived from a target capturing molecule, light (e.g., fluorescence) derived from an antibody bound to a bioparticle, a form of a bioparticle (e.g., morphology (an image acquired in bright field, in phase difference, or in dark field and a form characterized by image processing, particularly a form acquired by morphology processing), a state in which two or more bioparticles (cells or the like) are bound to each other, or the like), and a feature of a bioparticle predicted from morphological information about the bioparticle (e.g., a cell type or a cell state (living cell or dead cell)). The light, form, feature, and the like may be detected, for example, by an observation device including an objective lens, particularly a microscope device. The light, form, and feature may be detected, for example, by an imaging element or by a photodetector. On the basis of a result of detecting the light, form, feature, and the like in the detection step, a target capturing molecule to be cleaved in the linker cleavage step to be described later may be selected, or a bioparticle to be released from the surface 101 in the cleavage step S104 may be selected. For example, the imaging element may acquire an image of the surface 101 or an image of bioparticles captured on the surface 101, and a bioparticle to be released may be selected on the basis of the acquired image.

### (3-3-2) Linker Cleavage Step

The cleavage step S103 includes a linker cleavage step of cleaving the linker 1. By cleaving the linker 1, a bioparticle to which the target capturing molecule is bound is released from the surface 101. For example, as illustrated in Fig. 2C, by cleaving the linker 1 of the molecule 100, the molecule 100 is released from the surface 101, and accordingly, the bioparticle is also released from the surface 101.

In the cleavage step S103, the linker may be cleaved, for example, by stimulation such as chemical stimulation or photic stimulation. The photic stimulation is particularly suitable for selectively stimulating a specific narrow range.

The stimulation in the cleavage step S103 may be provided by a stimulation application device. The driving of the stimulation application device may be controlled, for example, by an information processing device such as a general-purpose computer. For example, the information processing device may drive the stimulation application device to selectively apply stimulation to a position of a bioparticle to be released. An example of the stimulation application device that may be adopted will be described below.

In order to selectively apply photic stimulation to a position of a cell, a light irradiation device may be used as the stimulation application device. The light irradiation device may be, for example, a digital micromirror device (DMD) or a liquid crystal display device. A selected position on the surface 101 can be irradiated with light by a micromirror constituting the DMD. The liquid crystal display device may be, for example, a reflective liquid crystal display, and a specific example thereof may include SXRD (Sony Corporation). By controlling a liquid crystal of the liquid crystal display device, a selected position on the surface 101 can be irradiated with light.

Alternatively, a liquid crystal shutter or a spatial light modulator may be used to selectively apply photic stimulation to a position of a cell. The liquid crystal shutter or the spatial light modulator can also apply the photic stimulation to a selected position.

A wavelength of light to be irradiated may be appropriately selected by a person skilled in the art depending on what type of linker the target capturing molecule includes.

The chemical stimulation may be applied by bringing a reagent for cleaving the linker 1 into contact with the surface 101. As described above, the reagent may be determined depending on the type of linker 1.

For example, in a case where the linker 1 includes a disulfide bond, the reagent may be a reducing agent capable of cleaving the bond, and examples thereof may include tris(2-carboxyethyl)phosphine (TCEP), dithiothreitol (DTT), and 2-mercaptoethanol. For example, in a case where TCEP is used, a reaction is performed at 50 mM for about 15 minutes.

For example, in a case where the linker 1 is a nucleic acid including a restriction endonuclease recognition sequence, the reagent may be a restriction endonuclease corresponding to each restriction endonuclease recognition sequence. 1 U of restriction endonuclease activity is an amount of endonuclease for completely degrading 1 µg of λDNA for one hour at 37°C in principle in 50 µl of each endonuclease reaction solution, and the amount of the endonuclease may be adjusted according to an amount of the restriction endonuclease recognition sequence.

At least one bioparticle released by the cleavage in the cleavage step S103 may be collected, for example, in a liquid such as a buffer. The liquid may be, for example, a hydrophilic liquid. The bioparticle-containing liquid obtained by the collection may be used in the isolation step S104 to be described later. In order to collect the released bioparticle, a fluid force caused when the liquid such as a buffer flows may be used, the bioparticle may be floated in the liquid under vibration, or the bioparticle may be floated in the liquid using the gravity or the like. The vibration may be, for example, vibration of the analysis substrate 102 or vibration of the liquid in which the bioparticle is contained. In addition, in order to float the bioparticle in the liquid using the gravity, the analysis substrate 102 may be moved such that the surface 101 faces the direction of gravity.

### (3-4) Isolation Step

In the isolation step S104, the bioparticle released from the surface 101 in the cleavage step is isolated in a microspace. By the isolation, the molecule 100 can be bound to a target substance included, for example, in the bioparticle. This makes it possible to associate the target substance, for example, with the barcode sequence included in the molecule 100. Using the barcode sequence information, it is possible to analyze a target substance, particularly a single cell.

The microspace may be, for example, a space in an emulsion particle or a space in a well. Preferably, in the isolation step S104, one bioparticle (particularly one bioparticle to which at least one molecule 100 is bound) is isolated in one emulsion particle or one well.

In an embodiment of the present technology, the isolation step S104 may include a determination step of determining whether to isolate a bioparticle into a microspace, and a particle isolation step of isolating the bioparticle determined to be isolated into the microspace in the determination step. This makes it possible to isolate only intended bioparticles. Therefore, for example, unintended bioparticles can be excluded from targets in the analysis step to be described later, thereby improving analysis efficiency.

The determination may be performed, for example, on the basis of light generated from a bioparticle (e.g., scattered light and/or autofluorescence), light generated from a substance bound to a bioparticle, or a morphological image. The substance bound to the bioparticle may be, for example, a target capturing molecule, or may be an antibody (particularly a fluorochrome-labeled antibody) bound to the bioparticle. The scattered light generated from the bioparticle may be, for example, forward scattered light and/or side scattered light. A doublet can be detected from a height and/or an area value of a signal acquired by detecting the scattered light. A single cell can be determined based on morphological image information. Whether the bioparticle is a dead cell can be determined from scattered light and/or a morphological image or fluorescence after the dead cell is dyed with a dead cell dyeing reagent, thereby removing the dead cell. In the present technology, the determination step may be performed immediately before the isolation step, thereby making it possible to reliably isolate only a single cell to which the barcode is assigned.

In another embodiment of the present technology, the particle isolation step may be performed without performing the determination step. By omitting the determination step, the number of steps in the analysis method according to the present technology can be reduced.

In another embodiment of the present technology, the determination step may be performed in the detection step described in section (3-3-1) above or the linker cleavage step described in section (3-3-2) above, rather than being performed in the isolation step. In this case, a bioparticle or a bioparticle population selected as a result of the determination in the detection step or the linker cleavage step is subjected to the particle isolation step to be described later. In this case, it is not necessary to use a device such as a cell sorter.

Hereinafter, the determination step and the particle isolation step will be described.

### (3-4-1) Determination Step

In the determination step, it is determined whether a bioparticle released in the step S104 is to be isolated into a microspace. As described above, the determination may be performed on the basis of light generated from the bioparticle or light generated from the substance bound to the bioparticle.

The determination step may include, for example, an irradiation step of irradiating the bioparticle with light and a detection step of detecting light generated by the irradiation.

The irradiation step may be performed, for example, by a light irradiation unit that irradiates the bioparticle with light. The light irradiation unit may include, for example, a light source that emits light. Furthermore, the light irradiation unit may include an objective lens that condenses light on the bioparticle. The light source may be appropriately selected by a person skilled in the art according to the purpose of analysis, and examples thereof may include a laser diode, an SHG laser, a solid-state laser, a gas laser, a highluminance LED, a halogen lamp, and a combination of two or more thereof. The light irradiation unit may include another optical element, if necessary, in addition to the light source and the objective lens.

The detection step may be performed, for example, by a detection unit that detects light generated from a bioparticle or a substance bound to the bioparticle. For example, the detection unit may detect light from the bioparticle or the substance bound to the bioparticle generated by the light irradiation unit irradiating light, e.g., scattered light and/or fluorescence. The detection unit may include, for example, a condenser lens that condenses light generated from the bioparticle and a detector. As the detector, a PMT, a photodiode, a CCD, a CMOS, or the like may be used, but the detector is not limited thereto. The detection unit may include another optical element, if necessary, in addition to the condenser lens and the detector. For example, the detection unit may further include a spectroscopic unit. Examples of optical components constituting the spectroscopic unit may include a grating, a prism, and an optical filter. The spectroscopic unit can detect, for example, light having a wavelength to be detected distinguishably from light having another wavelength. The detection unit may convert the detected light into an analog electric signal by photoelectric conversion. The detection unit may further convert the analog electric signal into a digital electric signal by AD conversion.

The determination step may be performed by a determination unit that performs determination processing as to whether to determine a bioparticle on the basis of the light detected in the detection step. The processing performed by the determination unit may be realized by, for example, an information processing device such as a general-purpose computer, particularly a processing unit included in the information processing device.

### (3-4-2) Particle Isolation Step

The isolation step S104 includes a particle isolation step of isolating a bioparticle into a microspace. In the present technology, the microspace may refer to a space having a dimension capable of accommodating one bioparticle to be analyzed. The dimension may be appropriately determined according to a factor such as a size of the bioparticle. The microspace may have a dimension capable of accommodating two or more bioparticles to be analyzed. In this case, however, not only one bioparticle but also two or more bioparticles may be accommodated in one microspace. The bioparticles in the microspace in which two or more bioparticles are accommodated may be excluded from targets to be disrupted in the disruption step to be described layer, or may be excluded from targets to be analyzed in the analysis step to be described later.

In addition, for example, a complex of a target capturing molecule and a target substance may be generated in the disruption step to be described later. A plurality of microspaces used in the present technology is preferably separated from each other so that the complex generated in one microspace does not shift to another microspace. Examples of the microspaces separated as described above can include spaces in wells and spaces in emulsion particles. That is, in a preferred embodiment of the present technology, the microspaces may be spaces in wells and spaces in emulsion particles. Hereinafter, an example of the particle isolation step in a case where the microspace is a space in a well and an example of the particle isolation step in a case where the microspace is a space in an emulsion particle will be individually described.

### (3-4-3) In Case Where Microspace Is Space in Well

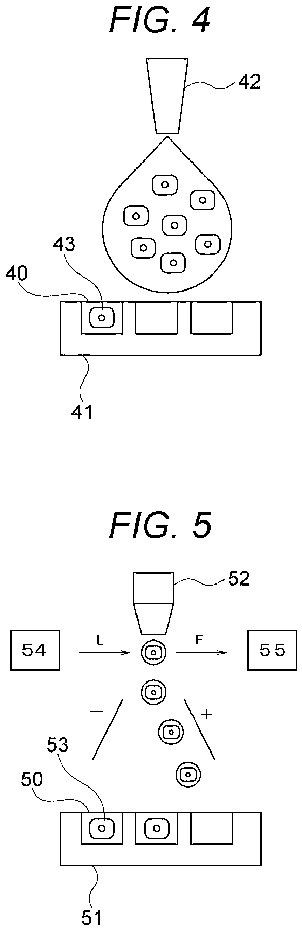

A schematic diagram of an example of a well used to perform the particle isolation step is illustrated in Fig. 4. As illustrated in Fig. 4, a plurality of wells 40, for example, each having a dimension capable of accommodating one bioparticle, may be formed in a surface of a substrate 41. By applying the bioparticle-containing liquid obtained in the cleavage step described in section (3-4) above to the surface of the substrate 41, for example, from an any type of nozzle 42, a bioparticle 43 is isolated into a space in the well 40 as illustrated in Fig. 4. In this way, one bioparticle may enter a space in one well, such that the bioparticle is isolated into the microspace.

When a liquid containing a plurality of bioparticles is applied to the substrate in which the wells are formed as in the example illustrated in Fig. 4, the particle isolation step may be performed without performing the determination step described in section (3-4-1) above.

In addition, when the determination step described in section (3-4-1) above is performed, for example, a device for putting one bioparticle in one well, such as a cell sorter or a single cell dispenser, may be used. For the device as well, a substrate (e.g., a plate) in which a plurality of wells is formed may be used to isolate bioparticles. As the device, a commercially available device may be used. The device may include, for example, a light irradiation unit that irradiates the bioparticles with light, a detection unit that detects light from a bioparticle, a determination unit that determines whether to put the bioparticle into a well on the basis of the detected light, and a distribution unit that distributes into the well the bioparticle determined to be put into the well.

The light irradiation unit and the detection unit perform the detection step, and the determination unit performs the determination step. The distribution unit includes, for example, a microfluidic chip having a nozzle that forms a droplet containing a bioparticle.

The device operates a position of the microfluidic chip according to a result of determination by the determination unit to put a droplet containing one bioparticle into a predetermined well. Alternatively, the device controls a direction in which a bioparticle-containing droplet discharged from the nozzle is headed, using a charge applied to the droplet according to a result of determination by the determination unit. This control makes it possible to put a droplet containing one bioparticle into a predetermined well. In this way, one bioparticle is distributed into one well.

For example, as illustrated in Fig. 5, a bioparticle-containing droplet is discharged from the nozzle 52 provided in the microfluidic chip of the device. The bioparticle contained in the droplet is irradiated with light (e.g., laser light L) by the light irradiation unit 54, and then, a detection step is performed by the detection unit 55 and light (fluorescence F) is detected. Then, the determination unit (not illustrated) performs a determination step on the basis of the detected light. Then, according to a determination result, the distribution unit controls a direction in which the droplet is headed using a charge applied to the droplet. This control makes it possible to collect a droplet containing an intended bioparticle in a predetermined well. Therefore, one bioparticle is distributed into one well.

By performing the determination step, it is possible to identify, for example, a cell population to which bioparticles according to a detection signal belong, a bioparticle to which a barcode is assigned, or a droplet containing a singlet bioparticle. This makes it possible to collect only droplets containing intended bioparticles. As a result, it is not necessary to exclude data in the analysis step to be described later, thereby improving analysis efficiency.

The number of wells provided in one substrate (plate) may be, for example, 1 to 1000, particularly 10 to 800, and more particularly 30 to 500, but the number of wells may be appropriately selected by a person skilled in the art.

### (3-4-4) In Case Where Microspace Is Space in

### Emulsion Particle

The emulsion particles may be produced, for example, using a microchannel. The device includes, for example, a channel through which a first liquid forming a dispersoid of an emulsion flows and a channel through which a second liquid forming a dispersion medium flows. The first liquid may contain bioparticles. The device further includes a region where the two liquids contact each other to form an emulsion. An example of the microchannel will be described below with reference to Fig. 6.

The microchannel illustrated in Fig. 6 includes a channel 61 through which a first liquid containing bioparticles flows, and channels 62-1 and 62-2 through which a second liquid flows. The first liquid forms emulsion particles (dispersoid), and the second liquid forms a dispersion medium of the emulsion. The channel 61 and the channels 62-1 and 62-2 join together, and the emulsion particles are formed at this junction point. Then, bioparticles 63 are isolated into the emulsion particles. For example, a size of the emulsion particles can be controlled by controlling a flow velocity in these channels.

The first liquid and the second liquid are immiscible with each other to form an emulsion. For example, the first liquid may be a hydrophilic liquid and the second liquid may be a hydrophobic liquid, or vice versa.

Furthermore, the microchannel illustrated in Fig. 6 may include a channel 64 for introducing a bioparticle-disrupting substance into the emulsion particles. By configuring the microchannel so that the channel 64 joins the channel 61 immediately before the junction point, it is possible to prevent the bioparticles from being disrupted by the bioparticle-disrupting substance before the emulsion particles are formed.

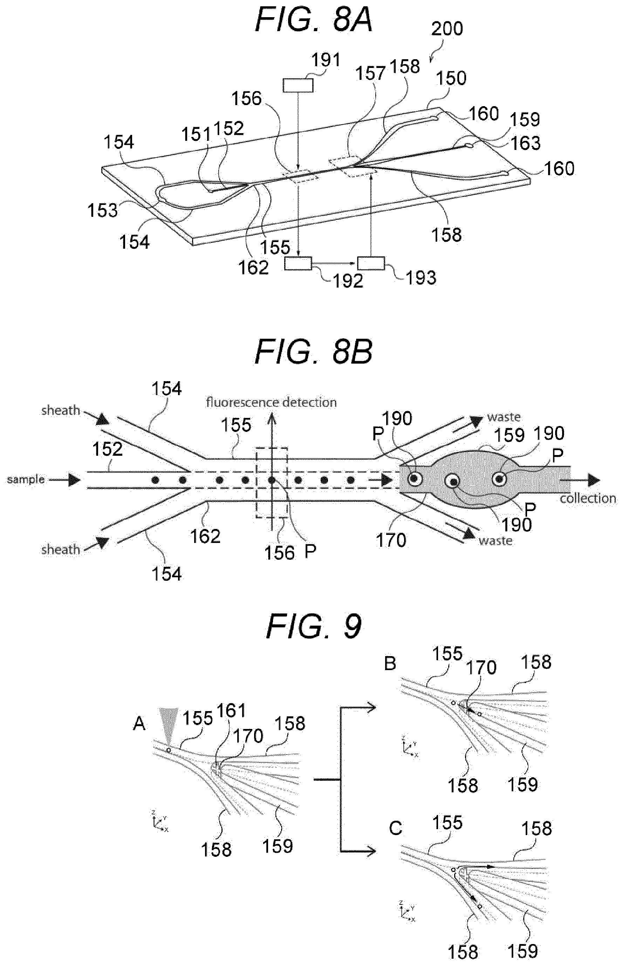

Next, an example of a device for more efficiently forming an emulsion containing emulsion particles each containing one bioparticle will be described with reference to Figs. 8A and 8B. The emulsion forming device makes it possible to isolate one bioparticle into one emulsion particle with very high probability, thereby reducing the number of empty emulsion particles. Moreover, the emulsion forming device makes it possible to increase probability that one bioparticle is isolated into one emulsion particle together with one barcode sequence.

Fig. 8A is an example of a microchip used for forming emulsion particles in the device. The microchip 150 illustrated in Fig. 8A includes a main channel 155 through which bioparticles flow and a collection channel 159 through which particles to be collected among the bioparticles are collected. The microchip 150 includes a particle sorting part 157. An enlarged view of the particle sorting part 157 is illustrated in Fig. 9. As illustrated in A of Fig. 9, the particle sorting part 157 includes a connection channel 170 that connects the main channel 155 and the collection channel 159 to each other. A liquid supply channel 161 capable of supplying a liquid to the connection channel 170 is connected to the connection channel 170. As described above, the microchip 150 has a channel structure including the main channel 155, the collection channel 159, the connection channel 170, and the liquid supply channel 161.

Fig. 8B is a schematic diagram illustrating how emulsion particles are formed and bioparticles are isolated into the formed emulsion particles in the microchip 150 illustrated in Fig. 8A.

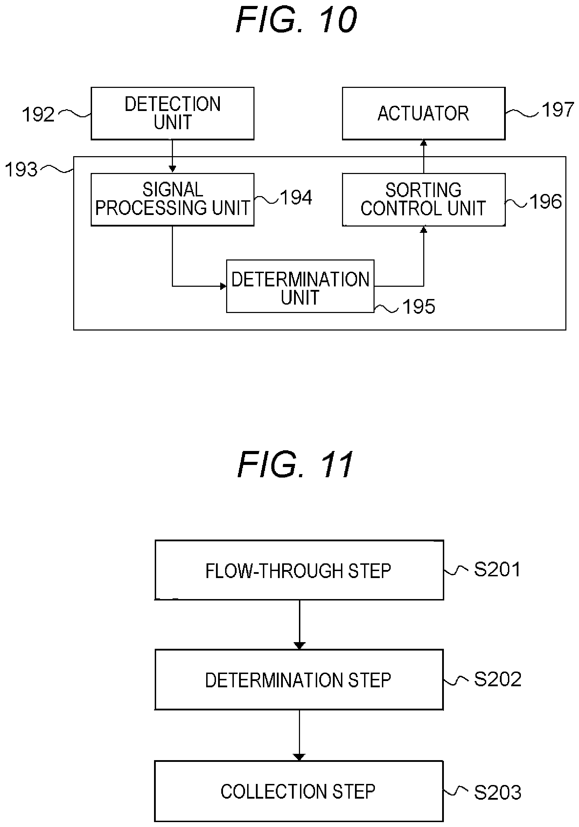

In addition, as illustrated in Fig. 8A, the microchip 150 constitutes a part of a bioparticle sorting device 200 including a light irradiation unit 191, a detection unit 192, and a control unit 193 in addition to the microchip. As illustrated in Fig. 10, the control unit 193 may include a signal processing unit 194, a determination unit 195, and a sorting control unit 196. The bioparticle sorting device 200 is used as the emulsion forming device described above.

As illustrated in Fig. 11, in order to form an emulsion containing emulsion particles each containing one intended bioparticle, for example, the following steps may be performed in the microchip 150: a flowthrough step S201 of causing a first liquid containing bioparticles to flow through the main channel 155, a determination step S202 of determining whether a bioparticle flowing through the main channel 155 is a particle to be collected, and a collection step S203 of collecting a particle to be collected into the collection channel 159. The determination step S202 corresponds to the determination step described in section (3-4-1) above. The collection step S203 corresponds to the particle isolation step described in section (3-4-2) above.

Each step will be described below.

### (Flow-Through Step)

In the flowing step S201, the first liquid containing bioparticles flows through the main channel 155. The first liquid flows from a junction portion 162 toward the particle sorting part 157 in the main channel 155. The first liquid may be a laminar liquid including a sample liquid containing bioparticles and a sheath liquid, and particularly, may be a laminar liquid in which the periphery of the sample liquid is surrounded by the sheath liquid. A channel structure for forming the laminar flow will be described below.

Note that the sheath liquid may contain, for example, a bioparticle-disrupting component, e.g., a cytolytic component. This makes it possible that the bioparticle-disrupting component incorporated into the emulsion particles disrupt the bioparticles in the emulsion particles in the disruption step to be described later. The cytolytic component may be a cytolytic enzyme, e.g., proteinase K. For example, after cells are captured in the emulsion particles containing proteinase K, the emulsion particles are placed at a predetermined temperature (e.g., 37°C to 56°C), for example, for 1 hour or less, particularly for less than 1 hour, to lyse the cells. Note that although the proteinase K is active even at 37°C or lower, the proteinase K may be incubated, for example, overnight, taking into account that the cytolytic property of the proteinase K decreases in a case where such a lower temperature is employed. Furthermore, the sheath liquid may contain a surfactant (e.g., SDS, Sarkosyl, Tween 20, or Triton X-100). The surfactant makes it possible to enhance the activity of the proteinase K.

Furthermore, the sheath liquid may not contain a bioparticle-disrupting component. In this case, bioparticles may be physically disrupted. As a physical disruption method, for example, optical treatment (e.g., optical lysis) or thermal treatment (e.g., thermal lysis) may be adopted. The optical treatment may be performed, for example, by forming plasma or cavitation bubbles in the emulsion particles by irradiating the particles with laser light. The thermal disruption of particles may be achieved by heating the emulsion particles.

The microchip 150 has a sample liquid inlet 151 and a sheath liquid inlet 153. From these inlets, the sample liquid containing bioparticles and the sheath liquid containing no bioparticles are introduced into a sample liquid channel 152 and a sheath liquid channel 154, respectively.

The microchip 150 has a channel structure in which the sample channel 152 through which the sample liquid flows and the sheath liquid channel 154 through which the sheath liquid flows join together at the junction portion 162 to become a main channel 155. The sample liquid and the sheath liquid join together at the junction portion 162, for example, to form a laminar flow in which the periphery of the sample liquid is surrounded by the sheath liquid. A schematic view of the formation of the laminar flow is illustrated in Fig. 8B. As illustrated in Fig. 8B, a laminar flow is formed so that the sample liquid introduced from the sample channel 152 is surrounded by the sheath liquid introduced from the sheath liquid channel 154.

Preferably, bioparticles are aligned substantially in a line in the laminar flow. For example, as illustrated in Fig. 8B, the bioparticles P may be aligned substantially in a line in the sample liquid. As described above, the channel structure according to the present technology forms a laminar flow including bioparticles flowing substantially in a line.

The laminar flow flows through the main channel 155 toward the particle sorting part 157. Preferably, the bioparticles flow side by side in the main channel 155. As a result, when light is irradiated in a detection region 156 to be described below, light generated by irradiating one microparticle with light and light generated by irradiating another microparticle with light can be easily distinguished.

### (Determination Step)

In the determination step S202, it is determined whether a bioparticle flowing through the main channel 155 is a particle to be collected. The determination may be performed by the determination unit 195. The determination unit 195 may perform the determination on the basis of light generated by the light irradiation unit 191 irradiating the bioparticle with light. An example of the determination step S202 will be described in more detail below.

In the determination step S202, the light irradiation unit 191 irradiates a bioparticle flowing through the main channel 155 (particularly the detection region 156) in the microchip 150 with light (e.g., excitation light), and the detection unit 192 detects light generated by irradiating the light. Based on a feature of the light detected by the detection unit 192, the determination unit 195 included in the control unit 193 determines whether the bioparticle is a particle to be collected. For example, the determination unit 195 may perform determination based on scattered light, determination based on fluorescence, or determination based on an image (e.g., one or more of a dark field image, a bright field image, and a phase difference image). In the collection step S203 to be described later, the control unit 193 controls a flow in the microchip 150 to collect a particle to be collected into the collection channel 159.

The light irradiation unit 191 irradiates a bioparticle flowing through the channel in the microchip 150 with light (e.g., excitation light). The light irradiation unit 191 may include a light source that emits light and an objective lens that condenses excitation light on a microparticle flowing in the detection region. The light source may be appropriately selected by a person skilled in the art according to the purpose of analysis, and examples thereof may include a laser diode, an SHG laser, a solid-state laser, a gas laser, a high-luminance LED, a halogen lamp, and a combination of two or more thereof. The light irradiation unit may include another optical element, if necessary, in addition to the light source and the objective lens.

### (Determination of Sorting Target Based on Fluorescence Signal and/or Scattered Light Signal)

In an embodiment of the present technology, the detection unit 192 detects scattered light and/or fluorescence generated from a microparticle by the light irradiation unit 191 irradiating light. The detection unit 192 may include a condenser lens that collects fluorescence and/or scattered light generated from the bioparticle and a detector. As the detector, a PMT, a photodiode, a CCD, a CMOS, or the like may be used, but the detector is not limited thereto. The detection unit 192 may include another optical element, if necessary, in addition to the condenser lens and the detector. For example, the detection unit 192 may further include a spectroscopic unit. Examples of optical components constituting the spectroscopic unit may include a grating, a prism, and an optical filter. The spectroscopic unit can detect, for example, light having a wavelength to be detected distinguishably from light having another wavelength. The detection unit 192 may convert the detected light into an analog electric signal by photoelectric conversion. The detection unit 192 may further convert the analog electric signal into a digital electric signal by AD conversion.

The signal processing unit 194 included in the control unit 193 may process a waveform of the digital electric signal obtained by the detection unit 192 to generate information (data) regarding a feature of light to be used for determination by the determination unit 105. As the information regarding the feature of the light, the signal processing unit 194 may acquire, for example, one, two, or three of a width of the waveform, a height of the waveform, and an area of the waveform from the waveform of the digital electric signal.
Furthermore, the information regarding the feature of the light may include, for example, a time at which the light is detected. The processing performed by the signal processing unit 194 described above may be performed particularly in an embodiment in which the scattered light and/or the fluorescence is detected.

On the basis of the light generated by irradiating the bioparticle flowing through the channel with light, the determination unit 195 included in the control unit 193 determines whether the bioparticle is a particle to be collected.

In the embodiment in which the scattered light and/or the fluorescence is detected, a waveform of a digital electric signal obtained by the detection unit 192 is processed by the control unit 193, and then, the determination unit 195 determines whether the bioparticle is a particle to be collected on the basis of information regarding a feature of light generated by the processing. For example, in a case where determination is made on the basis of scattered light, a feature of the bioparticle concerning its outer shape and/or internal structure may be specified, and it may be determined on the basis of the feature whether or not the bioparticle is a particle to be collected. Moreover, for example, by pretreating a bioparticle such as a cell in advance, it is also possible to determine whether the bioparticle is a particle to be collected on the basis of a feature similar to that used in flow cytometry. Furthermore, for example, by labeling a bioparticle such as a cell with an antibody or a dye (particularly a fluorochrome), it is also possible to determine whether the bioparticle is a particle to be collected on the basis of a feature of a surface antigen of the bioparticle.

### (Determination of Sorting Target Based on Bright Field Image and/or Phase Difference Image)

In another embodiment of the present technology, the detection unit 192 may acquire a bright field image and/or a phase difference image generated by the light irradiation unit 191 irradiating light. In this embodiment, for example, the light irradiation unit 191 may include a halogen lamp, and the detection unit 192 may include a CCD or a CMOS. For example, a bioparticle is irradiated with light by the halogen lamp, such that the CCD or CMOS may acquire a bright field image and/or a phase difference image of the irradiated bioparticle.

In the embodiment in which the bright field image and/or the phase difference image are acquired, the determination unit 195 included in the control unit 193 determines whether the bioparticle is a particle to be collected on the basis of the acquired bright field image and/or phase difference image. For example, it may be determined whether the bioparticle is a particle to be collected on the basis of one or a combination of two or more of a form, a size, and a color of the bioparticle (particularly the cell).

### (Determination of Sorting Target Based on Dark Field Image)

In another embodiment of the present technology, the detection unit 192 may acquire a dark field image generated by the light irradiation unit 191 irradiating light. In this embodiment, for example, the light irradiation unit 191 may include a laser light source, and the detection unit 192 may include a CCD or a CMOS. For example, a bioparticle is irradiated with light by the laser, such that the CCD or CMOS may acquire a dark field image (e.g., a fluorescence image) of the irradiated microparticle.

In the embodiment in which the dark field image is acquired, the determination unit 195 included in the control unit 193 determines whether the bioparticle is a particle to be collected on the basis of the acquired dark field image. For example, it may be determined whether the bioparticle is a particle to be collected on the basis of one or a combination of two or more of a form, a size, and a color of the bioparticle (particularly the cell).

In any of the "determination of sorting target based on fluorescence signal or/and scattered light signal", the "determination of sorting target based on bright field image", and the "determination of sorting target based on dark field image" described above, the detection unit 192 may be, for example, an imaging element in which a substrate equipped with a CMOS sensor and a substrate equipped with a digital signal processor (DSP) are laminated. By operating the DSP of the imaging element as a machine learning unit, the imaging element can operate as a so-called AI sensor. The detection unit 192 including the imaging element may determine whether the bioparticle is a particle to be collected, for example, on the basis of a learning model. Furthermore, the learning model may be updated in real time while the method according to the present technology is being performed. For example, the DSP may perform machine learning processing while resetting a pixel array unit of the CMOS sensor, while exposing the pixel array unit, or while reading out a pixel signal from each unit pixel of the pixel array unit. An example of the imaging element operating as an AI sensor may include an imaging device described in International Patent Application Publication No. 2018/051809. In a case where the AI sensor is used as an imaging element, raw data acquired from an image array is learned as it is, and thus, sorting-related determination processing is performed at a fast speed.

The determination may be performed, for example, based on whether the information regarding the feature of the light satisfies a criterion specified in advance. The criterion may be a criterion indicating that the bioparticle is a particle to be collected. The criterion may be appropriately set by a person skilled in the art, and may be, for example, a criterion related to a feature of light, such as a criterion used in the technical field pertaining to flow cytometry or the like.

Light may be irradiated toward one position in the detection region 156, or light may be irradiated toward each of a plurality of positions in the detection region 156. For example, the microchip 150 may be configured so that light is irradiated toward each of two different positions in the detection region 156 (that is, there are two positions irradiated with light in the detection region 156). In this case, it may be determined whether the bioparticle is a particle to be collected, for example, on the basis of light (e.g., fluorescence and/or scattered light) generated by irradiating the bioparticle with light at one position. Moreover, a velocity of the bioparticle in the channel can be calculated on the basis of a difference between a time at which light generated by irradiating the light at one position is detected and a time at which light generated by irradiating the light at the other position is detected. For the calculation, a distance between the two irradiation positions may be determined in advance, and the velocity of the bioparticle may be determined on the basis of the difference between the two detection times and the distance. Moreover, it is possible to accurately predict a time at which the bioparticle arrives at the particle sorting part 157 to be described below on the basis of the velocity. By accurately predicting the arrival time, it is possible to optimize a timing at which a flow entering the collection channel 159 is formed. Furthermore, in a case where a difference between a time at which a certain bioparticle arrives at the particle sorting part 157 and a time at which a bioparticle before or after the certain bioparticle arrives at the particle sorting part 157 is equal to or smaller than a predetermined threshold value, it can be determined that the certain bioparticle is not collected. In a case where a distance between a certain bioparticle and a bioparticle before or after the certain bioparticle is small, it is highly likely that the microparticle before or after the certain bioparticle is collected together with the certain bioparticle when the certain bioparticle is sucked. In a case where it is highly likely that the bioparticles are collected together, it is determined that the certain bioparticle is not collected, thereby making it possible to prevent the bioparticle before or after the certain bioparticle from being collected. As a result, it is possible to increase a ratio of intended bioparticles to the collected bioparticles. Specific examples of the microchip in which light is irradiated toward each of two different positions in the detection region 156 and the device including the microchip are described in, for example, Japanese Patent Application Laid-Open No. 2014-202573.

Note that the control unit 193 may control irradiation of light by the light irradiation unit 191 and/or detection of light by the detection unit 192. Furthermore, the control unit 193 may control driving of a pump for supplying a fluid into the microchip 150. The control unit 193 may include, for example, a hard disk in which a program for causing the device to execute the isolation step and an OS are stored, a CPU, and a memory. For example, the functions of the control unit 193 may be realized in a general-purpose computer. The program may be recorded in a recording medium such as a micro SD memory card, an SD memory card, or a flash memory. The program recorded in the recording medium may be read out by a drive (not illustrated) provided in the bioparticle sorting device 200, and then the control unit 193 may cause the bioparticle sorting device 200 to execute the isolation step according to the read-out program.

### (Collection Step)

In the collection step S203, the bioparticle determined as a particle to be collected in the determination step S202 is collected into the collection channel 159. In the collection step S203, the particle to be collected is collected, while being contained in a first liquid, into a second liquid immiscible with the first liquid in the collection channel. As a result, an emulsion containing the second liquid as a dispersion medium and the first liquid as a dispersoid can be formed in the collection channel 159, with one particle to be collected being contained in each emulsion particle of the emulsion. This makes it possible to isolate an intended bioparticle into a space in an emulsion particle.

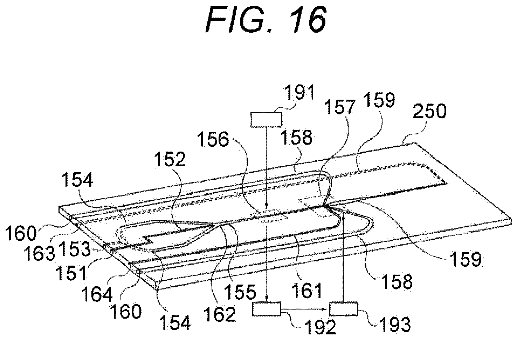

For example, as illustrated in Fig. 8B, a particle P to be collected is collected, while being contained in the first liquid indicated by white, in the second liquid indicated by gray in Fig. 16. As a result, emulsion particles 190 are formed, and one particle P to be collected is isolated into a space in one emulsion particle 190.

Hereinafter, the collection step will be described in more detail.

The collection step S203 is performed in the particle sorting part 157 of the microchip 150. In the particle sorting part 157, the laminar flow through the main channel 155 is divided into two waste channels 158. The particle sorting part 157 illustrated in Fig. 8A has two waste channels 158, but the number of branch channels is not limited to two. For example, the particle sorting part 157 may have one or a plurality of branched channels (e.g., two, three, or four branched channels). The branch channels may be configured to branch in a Y shape on one plane as in Fig. 8A, or may be configured to branch three-dimensionally.

Only when a particle to be collected flow into the particle sorting part 157, a flow from the main channel 155 into the collection channel 159 through the connection channel 170 is formed, such that the particle to be collected is collected into the collection channel 159. The enlarged view of the particle sorting part 157 is illustrated in Fig. 9. As illustrated in Fig. 9A, the main channel 155 and the collection channel 159 communicate with each other via the connection channel 170 that is coaxial with the main channel 155. As illustrated in Fig. 9B, the particle to be collected flows into the collection channel 159 through the connection channel 170. As illustrated in Fig. 9C\, microparticles that are not particles to be collected flows into the waste channels 158.

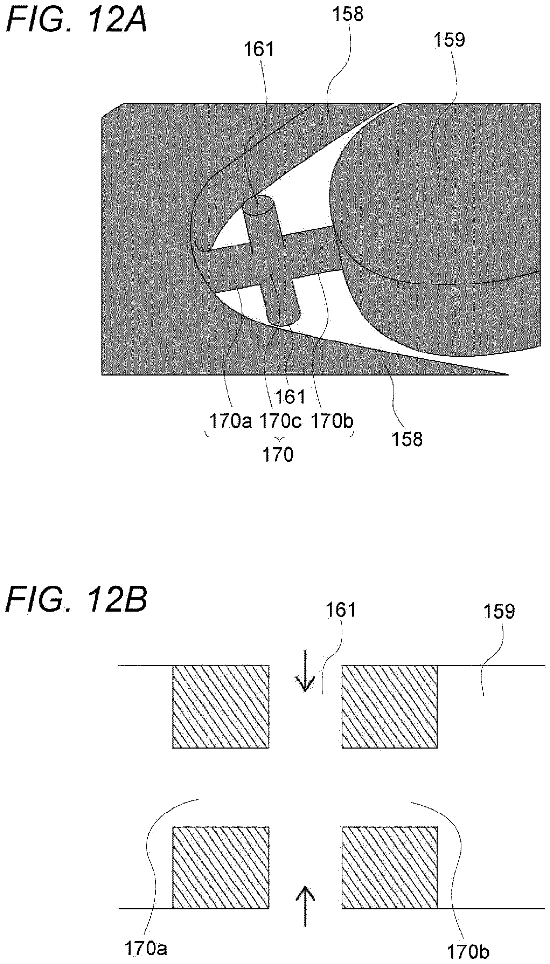

Figs. 12A and 12B are enlarged views of the vicinity of the connection channel 170. Fig. 12A is a schematic perspective view of the vicinity of the connection channel 170. Fig. 12B is a schematic cross-sectional view on a plane passing through a central line of the liquid supply channel 161 and a central line of the connection channel 170. The connection channel 170 includes a channel 170a close to the detection region 156 (hereinafter, also referred to as upstream connection channel 170a), a channel 170b close to the collection channel 159 (hereinafter, also referred to as downstream connection channel 170b), and a connection portion 170c between the connection channel 170 and the liquid supply channel 161. The liquid supply channel 161 is provided to be substantially perpendicular to an axis of the connection channel 170. In Figs. 12A and 12B, the two liquid supply channels 161 are provided to face each other at a substantially central position of the connection channel 170, but only one liquid supply channel may be provided.

A shape and a dimension of a cross section of the upstream connection channel 170a may be the same as a shape and a dimension of a cross section of the downstream connection channel 170b. For example, as illustrated in Figs. 12A and 12B, both the cross section of the upstream connection channel 120a and the cross section of the downstream connection channel 120b may be substantially circular while having the same dimension. Alternatively, both of these two cross sections may be quadrilateral (e.g., square or rectangular), while having the same dimension.

The second liquid is supplied from the two liquid supply channels 161 to the connection channel 170 as indicated by arrows in Fig. 12B. The second liquid flows from the connection portion 170c to both the upstream connection channel 170a and the downstream connection channel 170b.

In a case where the collection step is not performed, the second liquid flows as follows.

The second liquid flowing into the upstream connection channel 170a comes out of a surface of the connection channel 170 connected to the main channel 155, and then flows dividedly into the two waste channels 158. Since the second liquid comes out of the connected surface as described above, it is possible to prevent the first liquid and microparticles that do not need to be collected into the collection channel 159 from entering the collection channel 159 through the connection channel 170.

The second liquid flowing to the downstream connection channel 170b flows into the collection channel 159. As a result, the collection channel 159 is filled with the second liquid, and the second liquid serves as a dispersion medium, for example, for forming an emulsion.

In a case where the collection step is performed as well, the second liquid may be supplied from the two liquid supply channels 161 to the connection channel 170. However, due to a change in pressure in the collection channel 159, particularly by generating a negative pressure in the collection channel 159, a flow from the main channel 155 to the collection channel 159 through the connection channel 170 is formed. That is, a flow from the main channel 155 to the collection channel 159 through the upstream connection channel 170a, the connection portion 170c, and the downstream connection channel 170b in this order is formed. As a result, the particle to be collected is collected, while being surrounded by the first liquid, into the second liquid in the collection channel 159. By performing the collection step, for example, an emulsion may be formed in the collection channel 159 or in a container connected to a collection channel end 163, for example, via a channel.

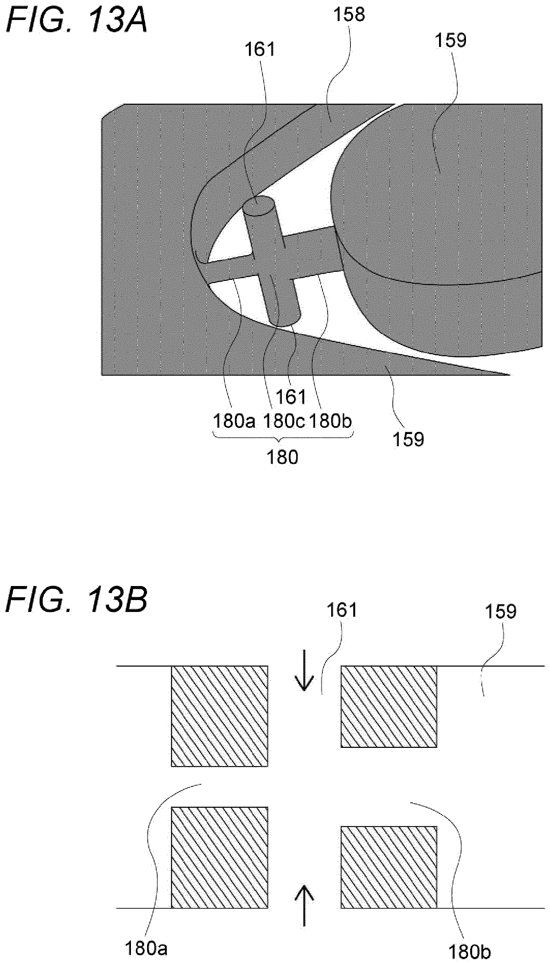

A shape and/or a dimension of a cross section of the upstream connection channel 120a may be different from a shape and/or a dimension of a cross section of the downstream connection channel 120b. Figs. 13A and 13B illustrate an example in which these two channels have different dimensions. As illustrated in Figs. 13A and 13B, a connection channel 180 includes a channel 180a close to the detection region 156 (hereinafter, also referred to as upstream connection channel 180a), a channel 180b close to the collection channel 159 (hereinafter, also referred to as downstream connection channel 180b), and a connection portion 180c between the connection channel 180 and the liquid supply channel 161. Both a cross section of the upstream connection channel 180a and a cross section of the downstream connection channel 180b have a substantially circular shape, but the cross section of the latter has a larger diameter than the cross section of the former. By making the diameter of the cross section of the latter larger than that of the former, it is possible to more effectively prevent a particle to be collected that has already been sorted into the collection channel 159 immediately after the microparticle is sorted by virtue of the negative pressure as described above from being discharged to the main channel 155 through the connection channel 180, as compared with the case where the diameters of the former and the latter are the same.

For example, in a case where both the cross section of the upstream connection channel 180a and the cross section of the downstream connection channel 180b are quadrilateral, the cross section of the latter has a larger area than the cross section of the former, thereby making it possible to more effectively prevent already-collected microparticles from being discharged to the main channel 155 through the connection channel 180 as described above.

In the collection step S203, a particle to be collected is collected into the collection channel through the connection channel due to a change in pressure in the collection channel 159. The collection may be performed, for example, by generating a negative pressure in the collection channel 159 as described above. The negative pressure may be generated when a wall defining the collection channel 159 is deformed, for example, by an actuator 197 (particularly a piezoelectric actuator) attached to the outside of the microchip 150. The flow entering the collection channel 159 may be formed by the negative pressure. In order to generate a negative pressure, for example, the actuator 197 may be attached to the outside of the microchip 150 so that the wall of the collection channel 159 may be deformed. The deformation of the wall makes it possible to change an inner space of the collection channel 159 and generate a negative pressure. The actuator 197 may be, for example, a piezoelectric actuator. When the particle to be collected is sucked into the collection channel 159, the sample liquid constituting the laminar flow or the sample liquid and the sheath liquid constituting the laminar flow may also flow into the collection channel 159. In this way, the particle to be collected is sorted in the particle sorting part 157 and collected into the collection channel 159.

The particle to be collected is collected, while being surrounded by the first liquid, into the second liquid immiscible with the first liquid in the collection channel 159. As a result, an emulsion containing the second liquid as a dispersion medium and the first liquid as a dispersoid is formed in the collection channel 159 as described above.

In order to prevent a bioparticle that is not a particle to be collected from entering the collection channel 159 through the connection channel 170, the connection channel 170 is provided with the liquid supply channel 161. The second liquid immiscible with the liquid (the sample liquid and the sheath liquid) flowing through the main channel 155 is introduced from the liquid supply channel 161 into the connection channel 170.

A flow from the connection channel 170 toward the main channel 155 is formed by a part of the second liquid introduced into the connection channel 170 to prevent bioparticles other than the particles to be collected from entering the collection channel 159. The second liquid flowing from the connection channel 170 toward the main channel 155 flows through the waste channels 158 similarly to the first liquid, rather than flowing into the main channel 155, due to the flow of the first liquid to the waste channels 158 through the main channel 155.

Note that the rest of the second liquid introduced into the connection channel 170 flows into the collection channel 159. As a result, the collection channel 159 may be filled with the second liquid.

The collection channel 159 may be filled with the second liquid immiscible with the first liquid. In order to fill the inside of the collection channel 159 with the second liquid, the second liquid may be supplied from the liquid supply channel 161 to the connection channel 170. By supplying the second liquid, the second liquid flows from the connection channel 170 to the collection channel 159, and accordingly, the collection channel 159 may be filled with the second liquid.

The liquid flowing into the waste channels 158 in laminar flow may be discharged out of the microchip at waste channel ends 160. The target particle collected into the collection channel 159 may be discharged out of the microchip at the collection channel end 161.

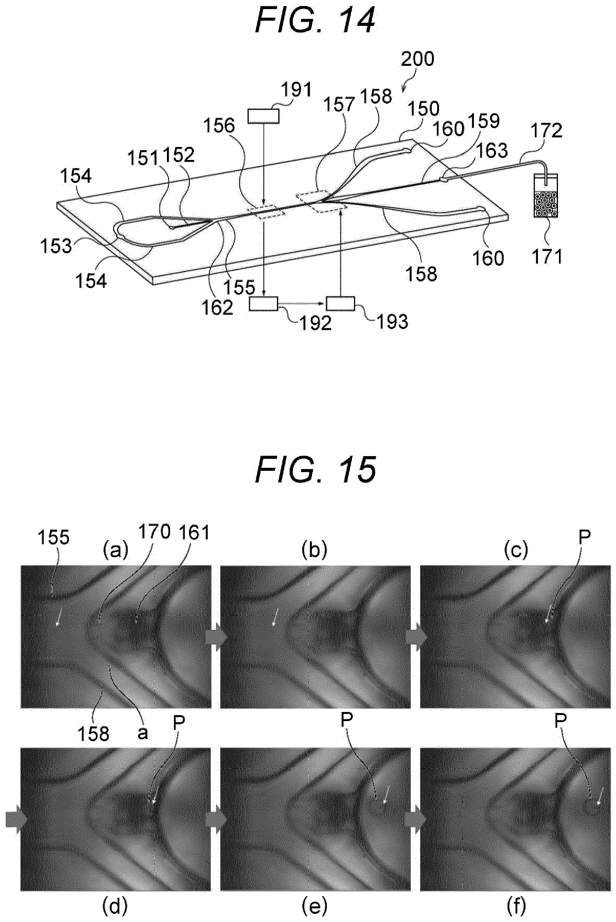

For example, as illustrated in Fig. 14, a container 171 may be connected to the collection channel end 163 via a channel such as a tube 172. As illustrated in Fig. 14, an emulsion containing the first liquid containing a particle to be collected as a dispersoid and the second liquid as a dispersion medium is collected into the container 171. In this way, according to an embodiment of the present technology, the bioparticle sorting device 200 may include a channel for collecting an emulsion containing a particle to be collected into the container.

Furthermore, when the collection operation is performed in a state where the collection channel end 163 is closed, a plurality of emulsion particles can be held in the collection channel 159. After the collection operation is completed, an assay such as single cell analysis can be continuously performed in the collection channel 159. For example, the disruption step to be described later may be performed in the collection channel 159. Then, a target substance may be bound to a target capturing molecule in the disruption step.

As described above, in the microchip used in the present technology, the main channel may be branched into the connection channel and the at least one waste channel. The at least one waste channel is a channel through which bioparticles other than particles to be collected flow.

Furthermore, as illustrated in Figs. 8A, 8B, and 9, in the microchip used in the present technology, the main channel, the connection channel, and the collection channel may be arranged in a straight line. In a case where these three channels are arranged in a straight line (particularly coaxially), the collection step can be performed more efficiently as compared with, for example, a case where the connection channel and the collection channel are arranged at an angle with respect to the main channel. For example, a suction amount required to guide a particle to be collected to the connection channel can be reduced.

Furthermore, as illustrated in Figs. 1 and 2, in the microchip used in the present technology, bioparticles flow toward the connection channel while being arranged substantially in a line in the main channel. Therefore, a suction amount can be reduced in the collection step.

Note that the channel configuration of the microchip used in the present technology is not limited to that illustrated in Fig. 8A. For example, in the microchip used in the present technology, two or more inlets and/or outlets, preferably all inlets and/or outlets, among inlets into which the liquids are introduced and outlets from which the liquids are discharged, may be formed in one surface. Fig. 16 illustrates a microchip in which inlets and outlets are formed as described above. In the microchip 250 illustrated in Fig. 16, all of the collection channel end 163 and the two branch channel ends 160 are formed on a surface where the sample liquid inlet 151 and the sheath liquid inlet 153 are formed. Moreover, an introduction channel inlet 164 for introducing liquid into the introduction channel 161 is formed in the surface. In this way, in the microchip 250 for sorting bioparticles, all of the inlets into which the liquids are introduced and the outlets from which the liquids are discharged are formed in one surface. This makes it easy to attach the chip to the bioparticle sorting device 200. For example, it is easy to connect the channels provided in the bioparticle sorting device 200 and the channels in the microchip 250 for sorting bioparticles to each other in a case where the inlets and the outlets are formed in one surface, as compared with a case where the inlets and/or the outlets are formed in two or more surfaces.

Note that, in Fig. 16, a part of the sheath liquid channel 154 is indicated by a dotted line. The portion indicated by the dotted line is disposed at a lower position than the sample liquid channel 152 indicated by a solid line (a position shifted in an optical axis direction indicated by an arrow), and these channels do not communicate with each other at a position where the channel indicated by the dotted line and the channel indicated by the solid line intersect. This description also applies to a part of the collection channel 159 indicated by a dotted line and the branch channel 158 intersecting the part of the collection channel 159.

Furthermore, in the present technology, the liquid supply channel supplies a liquid (particularly the second liquid) to the connection channel. As a result, a flow from a position at which the liquid supply channel and the connection channel are connected to each other toward the main channel is formed in the connection channel, thereby making it possible to prevent the liquid flowing through the main channel from entering the connection channel and prevent microparticles other than particles to be collected from flowing into the collection channel through the connection channel. When the collection step is performed, the first liquid containing one particle to be collected is collected into the second liquid in the collection channel through the connection channel, for example, due to a negative pressure generated in the collection channel, as described above. As a result, an emulsion particle containing one particle to be collected is formed in the second liquid.

Furthermore, in the present technology, a hydrophobic solution including a bioparticle determined to be a particle to be collected in the determination step is collected into the collection channel 159, for example, by driving the piezoelectric actuator at an appropriate timing (for example, at a time when the bioparticle arrives at the particle sorting part 157), to form an emulsion particle. In the determination step, by determining whether the particle is a particle to be collected, for example, using a peak signal and an area signal, it is also possible to determine whether there is one microparticle (singlet), a conjugate of two bioparticles (doublet), or a conjugate of three bioparticles (triplet). Therefore, it is possible to avoid forming emulsion particles each containing two or more bioparticles. Therefore, an emulsion particle containing one bioparticle can be formed with high probability and high efficiency. Furthermore, since it is possible to avoid forming an emulsion particle containing a conjugate of two or more bioparticles as described above, it is possible to omit an operation of removing a conjugate of two or more bioparticles before an operation of forming an emulsion, for example, by a cell sorter or the like.

### (Examples)

Using a microchip having a channel structure as illustrated in Fig. 8A, an emulsion particle containing one microparticle was formed as follows. Hereinafter, an operation for forming an emulsion particle will be described with reference to Fig. 15.

A piezoelectric element (a piezoelectric actuator) was attached to an outer surface of the microchip 150 (particularly an outer surface corresponding to a bulging portion close to a portion of the collection channel 159 connected to the connection channel 170) to change a volume in the collection channel 159.

A hydrophilic sample liquid containing beads having a diameter of 10 um was introduced from the sample liquid inlet 151 into the sample liquid channel 152, and a hydrophilic sheath liquid was introduced from the sheath liquid inlet 153 into the sheath liquid channel 154. The hydrophilic sample liquid and the hydrophilic sheath liquid introduced joined together at the junction portion 162, and then, a laminar flow in which the hydrophilic sample liquid was surrounded by the hydrophilic sheath liquid was formed. The laminar flow included beads arranged substantially in a line, and the laminar flow was formed in the main channel 155 toward the connection channel 170.

In addition to the introduction of the hydrophilic sample liquid and the hydrophilic sheath liquid, a hydrophobic liquid was supplied from the liquid supply channel 161 to the connection channel 170. By supplying the hydrophobic liquid from the liquid supply channel 161 to the connection channel 170, the laminar flow was prevented from entering the collection channel 159 through the connection channel 170, and the collection channel 159 was filled with the hydrophobic liquid.

When a bead passed through a position at which a laser beam with which the detection region 156 of the main channel 155 was irradiated, the bead was irradiated with the laser beam, and light was generated. The generated light was detected, and whether to collect each bead was determined on the basis of a feature of the detected light.

At a timing when the bead determined to be collected arrived at the vicinity of the connection channel 170, the piezoelectric element was driven to deform an inner cavity of the collection channel 159, and accordingly, the bead was collected into the collection channel 159 through the connection channel 170. The operations for the collection were as follows: (i) the collection channel 159 was deformed over 10 us to apply a negative pressure into the collection channel 159, (ii) the deformed state was maintained for 10 ps, and (iii) the negative pressure was eliminated over 10 us to restore the deformation. By repeating the above-described operations (i) to (iii), an emulsion containing emulsion particles each containing one bead in the hydrophobic liquid was formed in the collection channel 159. In the emulsion, the hydrophobic liquid was a dispersion medium and the emulsion particle containing the hydrophilic liquid was a dispersoid.

It is shown in Fig. 15 that an emulsion particle containing one bead is formed in the collection channel 159 by performing the above-described operations (i) to (iii). Fig. 15 will be described below.
(a) of Fig. 15 is a photograph showing a state in which the bead (indicated by a white arrow) flows toward the connection channel 170. At a time point of Fig. 15, the hydrophilic liquid flows in the main channel 155 toward the particle sorting part 157, and then flows to the two waste channels 158 branched from the main channel 155 without flowing to the connection channel 170. The hydrophobic liquid is supplied from the liquid supply channel 161 to the connection channel 170, and flows to both the main channel 155 and the collection channel 159. The hydrophobic liquid having flowed into the main channel 155 flows dividedly into the two waste channels 158 immediately after leaving the connection channel 170 due to the flow of the hydrophilic liquid (the hydrophobic liquid flows along walls of the waste channels 158 in the vicinity of the connection channel shown in a of Fig. 15).
(b) of Fig. 15 is a photograph at a time point when the bead further approaches the connection channel. At this time point, an operation for collecting the bead is started. That is, deforming the inner cavity of the collection channel 159 is started by driving the piezoelectric element.
(c) of Fig. 15 is a photograph showing a state in which the hydrophilic liquid is advancing toward the collection channel 159. It can be seen from the photograph that, at a time point of Fig. 15, the bead is surrounded by the hydrophilic liquid, and the hydrophilic liquid is surrounded by the hydrophobic liquid. That is, an emulsion particle P containing one bead is formed.
(d) of Fig. 15 is a photograph immediately before the emulsion particle P enters the collection channel 159 from the connection channel 170.
(e) of Fig. 15 is a photograph immediately after the emulsion particle P enters the collection channel 159. It can be confirmed that one bead is contained in the emulsion particle P.
(f) of Fig. 15 is a photograph showing that the emulsion particle P has flowed further downstream in the marine channel 159.

As described above, an emulsion particle containing one bead has been formed. It can be seen from this result that the bioparticle sorting device 200 can form an emulsion containing a high proportion of emulsion particles each containing one bioparticle.

### (3-5) Disruption Step

In the disruption step S105, the bioparticle is disrupted in the microspace. By performing the disruption, for example, the molecule 100 bound to the bioparticle via the bioparticle capturing part 7 may be dissociated from the bioparticle.

Note that, among the components of the disrupted bioparticle, a component bound to the bioparticle capturing part 7 may be bound to the molecule 100 via the bioparticle capturing part 7 even after the disruption.

In the disruption step S105, a target substance constituting the bioparticle or a target substance bound to the bioparticle may be captured by the target substance capturing part 6 included in the molecule 100. As a result, a complex of the molecule 100 and the target substance is formed, such that the target substance can be associated with a barcode sequence included in the molecule 100 in the analysis step to be described later. The complex formed in this way is analyzed in the analysis step to be described later.

The disruption step S105 is executed preferably while the isolated state of the bioparticle in the microspace is maintained. As a result, a complex of the molecule 100 and the target substance is efficiently formed. Furthermore, it is possible to prevent the target substance from being bound to a target capturing molecule outside the microspace.

In a case where the microspace refers to a space in an emulsion particle, the maintaining of the isolated state may mean that the emulsion particle is maintained, and particularly, may mean that the emulsion particle is not disrupted.

In a case where the microspace refers to a space in a well, the maintaining of the isolated state may mean that a component in the well (particularly a bioparticle in the well, a component of the bioparticle, and a target capturing molecule) remains in the well, and may further mean that a component in another well (particularly a bioparticle in another well, a component of the bioparticle, and a target capturing molecule in another well) does not invade the well.

In addition, when a nucleic acid-bound antibody is bound to a bioparticle as described in the section "(3-2) Capture Step" above, the nucleic acid-bound antibody is dissociated from the bioparticle in the disruption step S105. Then, the nucleic acid-bound antibody is bound to the target substance, such that a complex of the nucleic acid-bound antibody and the target substance may be formed. For example, a poly A sequence constituting the first nucleic acid 21 may be bound to an mRNA in a bioparticle as a target substance. Since the second nucleic acid 2 including an antibody barcode sequence is bound to the first nucleic acid 21, the target substance can be associated with the antibody barcode sequence. The complex formed in this way is analyzed in the analysis step to be described later.

The disruption step S105 may be performed by chemically or physically disrupting the bioparticle.

In order to chemically disrupt the bioparticle, a bioparticle-disrupting substance may be brought into contact with the bioparticle in the microspace. The bioparticle-disrupting substance may be appropriately selected by a person skilled in the art depending on the type of bioparticle. In a case where the bioparticle is a cell or an exosome, for example, a lipid bilayer membrane disrupting component may be used as the bioparticle disrupting substance, and particularly, a surfactant, an alkali component, an enzyme, or the like may be used. As the surfactant, an anionic surfactant, a nonionic surfactant, an amphoteric surfactant, or a cationic surfactant may be used. Examples of the anionic surfactant may include sodium dodecyl sulfate (SDS) and sodium lauroyl sarcosine. Examples of the nonionic surfactant may include Triton X-100, Triton X-114, Tween 20, Tween 80, NP-40, Brij-35, Brij-58, octyl glucoside, octylthioglucoside, and octylphenoxypolyethoxyethanol. Examples of the amphoteric surfactant may include CHAPS and CHAPSO. Examples of the cationic surfactant may include cetyltrimethylammonium bromide (CTAB). In addition, examples of the alkali component may include OH⁻ ions. In addition, examples of the enzyme may include proteinase K, streptolidine, lysozyme, lysostaphin, zymolase, cellulase, glycanase, and protease. The type of enzyme may be appropriately selected, for example, depending on the type of cell (animal cell, plant cell, bacteria, yeast, or the like).

In a case where the microspace is a space in a well, the disruption step may be performed, for example, by adding a bioparticle-disrupting substance to each well. Since the wells are isolated from each other, the components in the wells are maintained in the wells even though disruption occurs.

In a case where the microspace is a space in an emulsion particle, for example, a bioparticle-disrupting substance may be introduced into the emulsion particle simultaneously with the formation of the emulsion particle. Then, after the emulsion particle is formed, a step of disrupting the bioparticle by the bioparticle-disrupting substance may be performed.

In order to physically disrupt the bioparticle, physical stimulation for disrupting the bioparticle may be applied to the bioparticle. As a treatment for applying the physical stimulation to the bioparticle, for example, an optical treatment, a thermal treatment, an electrical treatment, an acoustic treatment, a freeze-thaw treatment, or a mechanical treatment may be adopted. Through such a treatment, a cell or an exosome can be disrupted. Examples of the optical treatment may include plasma formation or cavitation bubble formation by laser light irradiation. Examples of the thermal treatment may include a heat treatment. Examples of the acoustic treatment may include sonication using ultrasonic waves. Examples of the mechanical treatment may include a treatment using a homogenizer or a bead mill. The physical disruption of the bioparticle through such a treatment can be applied to both the case where the microspace is a space in a well and the case where the microspace is a space in an emulsion particle. In a case where the microspace is a space in an emulsion particle, an optical treatment, a thermal treatment, an electrical treatment, and a freeze-thaw treatment are particularly suitable among the above-described treatments. In order to disrupt the bioparticle while preventing the emulsion particle from being disrupted through the acoustic treatment, for example, a surfactant may be added into the emulsion particle, and moreover, a concentration of the surfactant may be adjusted.

In the disruption step S105, the collection sequence part 2 included in the molecule 100 may be used. A target substance may be bound to the molecule 100, and the target substance can be efficiently collected by using the collection sequence part 2. That is, the disruption step S105 includes a step of collecting the molecule 100 (particularly the target substance bound to the molecule 100) using the collection sequence part 2.

### (3-6) Analysis Step

In the analysis step S106, a bioparticle is analyzed. Particularly, in the analysis step S106, a target substance is analyzed. An analysis method may be determined, for example, depending on the type of target substance and the purpose of analysis.

In the present technology, in the analysis step S106, the barcode sequence and the target substance may be associated with each other. More specifically, the barcode sequence may be associated with a result of analyzing the target substance. The analysis result may include, for example, sequence information and/or amount information about the target substance. In the isolation step, one bioparticle is isolated into one microspace, and a plurality of molecules 100 capturing the one bioparticle all have the same barcode sequence. Therefore, all analysis results associated with one barcode sequence by performing the above-described association are derived from one bioparticle, and can be used for analyzing the one bioparticle.

In the analysis step S106, since a molecule 100 including a barcode sequence is bound to a target substance in the disruption step, even when different bioparticles present in a plurality of microspaces are collectively analyzed, an analysis result can be associated with each bioparticle on the basis of the barcode sequence.

For example, in a case where the microspace is a space in a well, each bioparticle disruption product in the well may be separately analyzed, or bioparticle disruption products in a plurality of wells may be collected as one sample, and collectively analyzed as the one sample. In the case of the former, it is easy to associate a bioparticle with an analysis result. In the case of the latter as well, since a target substance in each bioparticle disruption product forms a complex with a molecule 100 including a barcode sequence (or a nucleic acid-bound antibody including an antibody barcode sequence), each bioparticle can be associated with an analysis result thereof.

In a case where the microspace is a space in an emulsion particle, a plurality of emulsion particles may be collectively analyzed, and for example, the obtained emulsion may be collectively analyzed in its entirety. Since a target substance in each bioparticle disruption product forms a complex with a molecule 100 including a barcode sequence (or a nucleic acid-bound antibody including an antibody barcode sequence), each bioparticle can be associated with an analysis result thereof. As a result, analysis efficiency can be improved.

In the analysis step S106, for example, a bioparticle is analyzed. The analysis may be performed, for example, on a complex of a molecule 100 and a target substance and/or a complex of a nucleic acid-bound antibody and a target substance formed in the disruption step S105. Since the molecule 100 and the nucleic acid-bound antibody include a barcode sequence and an antibody barcode sequence, respectively, it is possible to identify a bioparticle from which the target substance is derived on the basis of the barcode sequence.

In a case where the target substance has a base sequence, for example, in a case where the target substance is an RNA (particularly an mRNA) or a DNA, the base sequence of the target substance may be subjected to sequencing processing in the analysis step S106. The sequencing processing may be performed, for example, by a next-generation sequencer.

The analysis in the analysis step S106 may be performed, for example, using the amplification sequence part 3 included in the molecule 100. For example, the analysis step S106 includes a step of amplifying a nucleic acid using the amplification sequence part 3. As a result, for example, a nucleic acid (particularly an mRNA) bound to the molecule 100 may be amplified. Then, information regarding the nucleic acid can be acquired by performing sequencing processing on the sequence of the nucleic acid.

Furthermore, by performing the amplification, the barcode sequence included in the barcode sequence part may also be amplified. As a result, the information regarding the nucleic acid can be associated with the barcode sequence included in the molecule 100, and further, can be associated with the bioparticle.

The analysis step S106 may be performed using an analysis device 120 as illustrated in Fig. 3F. The analysis device 120 may be, for example, a device that performs sequencing processing on the complex. The sequencing processing may be performed, for example, in a case where the target substance is a nucleic acid, particularly a DNA or an RNA, and more particularly an mRNA. The sequencing processing may be performed by a sequencer, or may be performed by a next-generation sequencer or a Sanger method-based sequencer. In order to comprehensively analyze a plurality of bioparticles (particularly a cell population) at a higher speed, the sequencing processing may be performed by a next-generation sequencer.

In order to perform sequencing processing in the analysis step S106, the analysis step may further include a step of preparing a nucleic acid (e.g., a cDNA) to be subjected to sequencing processing and a step of purifying the nucleic acid. Through these preparation and purification steps, for example, a library for performing next-generation sequencing processing may be prepared.

In the preparation of the library, the collection sequence part 2 may be used. A molecule 100 to which a target substance is bound may be collected using a bead on which a nucleic acid having a sequence complementary to the nucleic acid sequence included in the collection sequence part 2 is immobilized.

The preparation step may include, for example, a cDNA synthesis step of synthesizing a cDNA from an mRNA. Furthermore, the preparation step may include an amplification step of amplifying the synthesized cDNA. After the preparation step, a purification step of purifying the nucleic acid obtained in the preparation step may be performed. The purification step may include, for example, a treatment for degrading components other than the nucleic acid using an enzyme such as proteinase K. Furthermore, in the purification step, a nucleic acid collection treatment may be performed. In the nucleic acid collection treatment, for example, a commercially available reagent for purifying nucleic acids may be used, and examples thereof may include magnetic beads such as AMPure XP. Note that, in the purification step, an intracellular dsDNA can also be collected, but the dsDNA can be prevented from being sequenced in the sequencing processing.

For example, an adaptor sequence for sequencing processing (particularly for next-generation sequencing processing) is included in a target capturing molecule, thereby making it possible to sequence only a nucleic acid including the adaptor sequence.

In the analysis step S106, components may be analyzed for each bioparticle on the basis of a sequencing processing result. For example, in the analysis step S106, a sequence of an mRNA included in a cell and/or the number of copies of each mRNA may be determined for each bioparticle. Furthermore, in the analysis step S106, the type and/or the number of antigens or the type and/or the number of transcription factors may be determined for each bioparticle. Such analysis of the components for each bioparticle may be performed on the basis of a barcode sequence among sequences determined by the sequence processing. For example, sequences including the same barcode sequence are selected from among a large number of sequences determined by the sequence processing. The sequences including the same barcode sequence are based on target capturing molecules taken up by one cell. Therefore, the analysis of the components for each barcode sequence means that components are analyzed for each bioparticle.

### 2. Second Embodiment (Bioparticle Analysis System)

The present technology also provides a bioparticle analysis system including a capture kit for capturing a bioparticle, the capture kit having a surface on which a molecule including a bioparticle capturing part, a barcode sequence, and a cleavable linker is immobilized via the linker. The bioparticle analysis system may further include a linker cleavage device that cleaves the linker to release the bioparticle from the surface, and/or an isolation device that forms or has a microspace into which the bioparticle released from the surface is isolated.

The capture kit may include, for example, a substrate having a surface on which a molecule including a bioparticle capturing part, a barcode sequence, and a cleavable linker is immobilized via the linker. The substrate may be an analysis substrate 102 as described in section (3-1) of "1. First Embodiment (Bioparticle Analysis Method)" above, and the description thereof also applies to the present embodiment.

The linker cleavage device may be, for example, a stimulation application device as described in section (3-3-2) of "1. First Embodiment (Bioparticle Analysis Method)" above, and the description thereof also applies to the present embodiment.

The bioparticle analysis system according to the present technology may further include an imaging element that images the surface 101 or the bioparticle captured on the surface 101. The linker cleavage device may apply stimulation so that a bioparticle selected on the basis of an image acquired by the imaging element is dissociated from the surface 101.

The isolation device may include a well as described in section (3-4-3) of "1. First Embodiment (Bioparticle Analysis Method)" above, and may include, for example, a substrate (e.g., a plate) including the well. Furthermore, the isolation device may include a nozzle that applies a bioparticle-containing droplet to the well as described in section (3-4-3) above. The isolation device may include a device that puts one bioparticle in one well, such as a cell sorter or a single cell dispenser, as described in section (3-4-3) above.

Alternatively, the isolation device may include a microchannel as described in section (3-4-4) of "1. First Embodiment (Bioparticle Analysis Method)" above, or may include a microchip 150 as described in section (3-4-4) above. The isolation device may be a bioparticle sorting device 200 as described in section (3-4-4) above.

The bioparticle analysis system according to the present technology may further include an analysis device that analyzes a target substance constituting the bioparticle or a target substance bound to the bioparticle. The analysis device may be a device that performs analysis as described in section (3-6) of "1. First Embodiment (Bioparticle Analysis Method)" above, e.g., a sequencer.

The bioparticle analysis system according to the present technology may perform the bioparticle analysis method described in "1. First Embodiment (Bioparticle Analysis Method)" above. Therefore, the description regarding the bioparticle analysis method also applies to the present embodiment.

For example, the bioparticle analysis system according to the present technology may perform a capture step S102, a cleavage step S103, and an isolation step S104 as described above. Furthermore, the bioparticle analysis system according to the present technology may perform a disruption step S105 after the isolation step S104, and may further perform a part of an analysis step S106, for example, a sample preparation step of preparing a sample to be subjected to the analysis step S106, after the disruption step S105. For example, in a case where the target molecule is an mRNA, the sample preparation step may include a cDNA synthesis step of synthesizing a cDNA from an mRNA.

For example, the system may be configured to automatically perform the capture step S102, the cleavage step S103, and the isolation step S104, may particularly be configured to automatically perform the capture step S102, the cleavage step S103, the isolation step S104, and the disruption step S105, and may more particularly be configured to automatically perform the capture step S102, the cleavage step S103, the isolation step S104, the disruption step S105, and the analysis step S106 (or the sample preparation step or the like as a part thereof).

### 3. Third Embodiment (Surface)

The present technology also provides a surface on which a molecule including a bioparticle capturing part, a barcode sequence, and a cleavable linker is immobilized via the linker. The surface is a surface as described in "1. First Embodiment (Bioparticle Analysis Method)" above, and the description thereof also applies to the present embodiment. The present technology also provides a substrate having the surface. The surface and the substrate may be those used in "1. First Embodiment (Bioparticle Analysis Method)" above.

Note that the present technology can also have the following configurations.
[1] A bioparticle analysis method including:
   a capture step of capturing a bioparticle on a surface, on which a molecule including a bioparticle capturing part, a barcode sequence, and a cleavable linker is immobilized via the linker, via the bioparticle capturing part;
   a cleavage step of cleaving the linker to release the bioparticle from the surface; and
   an isolation step of isolating the bioparticle into a microspace.
[2] The bioparticle analysis method according to [1], in which in the capture step, a plurality of molecules bound to one bioparticle have the same barcode sequence.
[3] The bioparticle analysis method according to [1] or [2], further including a disruption step of disrupting the bioparticle in the microspace.
[4] The bioparticle analysis method according to [3], in which in the disruption step, the molecule is dissociated from the bioparticle.
[5] The bioparticle analysis method according to [3] or [4], in which the molecule further includes a target substance capturing part, and
   in the disruption step, a target substance constituting the bioparticle or a target substance bound to the bioparticle is captured by the target substance capturing part.
[6] The bioparticle analysis method according to [5], further including an analysis step of analyzing the target substance after the disruption step.
[7] The bioparticle analysis method according to [6], in which in the analysis step, the barcode sequence is associated with the target substance.
[8] The bioparticle analysis method according to [6] or [7], in which the target substance has a base sequence, and
   in the analysis step, sequencing processing is performed on the base sequence of the target substance.
[9] The bioparticle analysis method according to any one of [1] to [8], in which in the cleavage step, a bioparticle to be released from the surface is selected on the basis of a label of the bioparticle or a label of the molecule.
[10] The bioparticle analysis method according to any one of [1] to [9], in which in the cleavage step, the linker is cleaved by chemical stimulation or photic stimulation.
[11] The bioparticle analysis method according to any one of [1] to [10], in which in the cleavage step, the captured state of the bioparticle is maintained by the bioparticle capturing part.
[12] The bioparticle analysis method according to any one of [1] to [11], in which in the cleavage step, only a selected bioparticle is released from the surface.
[13] The bioparticle analysis method according to any one of [1] to [12], in which the microspace is a space in an emulsion particle or a space in a well.
[14] The bioparticle analysis method according to any one of [1] to [13], further including a determination step of determining whether to isolate a bioparticle released from the surface in the cleavage step into a microspace.
[15] The bioparticle analysis method according to [14], in which in the determination step, the determination is performed on the basis of light generated by irradiating the bioparticle with light.
[16] The bioparticle analysis method according to any one of [1] to [15], in which in the capture step, the bioparticle and the bioparticle capturing part are bound to each other in a specific or non-specific manner.
[17] The bioparticle analysis method according to any one of [1] to [16], in which the capture step includes an incubation step for binding the bioparticle and the bioparticle capturing part to each other.
[18] A bioparticle analysis system including:
   a capture kit configured to capture a bioparticle, the capture kit having a surface on which a molecule including a bioparticle capturing part, a barcode sequence, and a cleavable linker is immobilized via the linker;
   a linker cleavage device that cleaves the linker to release the bioparticle from the surface; and
   an isolation device that forms or has a microspace into which the bioparticle released from the surface is isolated.
[19] The bioparticle analysis system according to [18], further including an analysis device that analyzes a target substance constituting the bioparticle or a target substance bound to the bioparticle.

### REFERENCE SIGNS LIST

- 100: Molecule
- 101: Surface
- 102: Substrate

## Claims

1. A bioparticle analysis method comprising:
a capture step of capturing a bioparticle on a surface, on which a molecule including a bioparticle capturing part, a barcode sequence, and a cleavable linker is immobilized via the linker, via the bioparticle capturing part;
a cleavage step of cleaving the linker to release the bioparticle from the surface; and
an isolation step of isolating the bioparticle into a microspace.

2. The bioparticle analysis method according to claim 1, wherein in the capture step, a plurality of molecules bound to one bioparticle have the same barcode sequence.

3. The bioparticle analysis method according to claim 1, further comprising a disruption step of disrupting the bioparticle in the microspace.

4. The bioparticle analysis method according to claim 3, wherein in the disruption step, the molecule is dissociated from the bioparticle.

5. The bioparticle analysis method according to claim 3, wherein the molecule further includes a target substance capturing part, and
in the disruption step, a target substance constituting the bioparticle or a target substance bound to the bioparticle is captured by the target substance capturing part.

6. The bioparticle analysis method according to claim 5, further comprising an analysis step of analyzing the target substance after the disruption step.

7. The bioparticle analysis method according to claim 6, wherein in the analysis step, the barcode sequence is associated with the target substance.

8. The bioparticle analysis method according to claim 6, wherein the target substance has a base sequence, and
in the analysis step, sequencing processing is performed on the base sequence of the target substance.

9. The bioparticle analysis method according to claim 1, wherein in the cleavage step, a bioparticle to be released from the surface is selected on a basis of a label of the bioparticle or a label of the molecule.

10. The bioparticle analysis method according to claim 1, wherein in the cleavage step, the linker is cleaved by chemical stimulation or photic stimulation.

11. The bioparticle analysis method according to claim 1, wherein in the cleavage step, the captured state of the bioparticle is maintained by the bioparticle capturing part.

12. The bioparticle analysis method according to claim 1, wherein in the cleavage step, only a selected bioparticle is released from the surface.

13. The bioparticle analysis method according to claim 1, wherein the microspace is a space in an emulsion particle or a space in a well.

14. The bioparticle analysis method according to claim 1, further comprising a determination step of determining whether to isolate a bioparticle released from the surface in the cleavage step into a microspace.

15. The bioparticle analysis method according to claim 14, wherein in the determination step, the determination is performed on a basis of light generated by irradiating the bioparticle with light.

16. The bioparticle analysis method according to claim 1, wherein in the capture step, the bioparticle and the bioparticle capturing part are bound to each other in a specific or non-specific manner.

17. The bioparticle analysis method according to claim 1, wherein the capture step includes an incubation step for binding the bioparticle and the bioparticle capturing part to each other.

18. A bioparticle analysis system comprising:
a capture kit configured to capture a bioparticle, the capture kit having a surface on which a molecule including a bioparticle capturing part, a barcode sequence, and a cleavable linker is immobilized via the linker;
a linker cleavage device that cleaves the linker to release the bioparticle from the surface; and
an isolation device that forms or has a microspace into which the bioparticle released from the surface is isolated.

19. The bioparticle analysis system according to claim 18, further comprising an analysis device that analyzes a target substance constituting the bioparticle or a target substance bound to the bioparticle.
